# EUROPEAN PATENT SPECIFICATION

(11) **EP 4 263 677 B1**
(45) Date of publication and mention of the grant of the patent: **30.10.2024**
(21) Application number: 21843847.1
(22) Date of filing: 17.12.2021
(51) Int. Cl.: C08H 7/00, C07D 307/50

(54) **METHODS AND SYSTEMS FOR PRODUCTION OF FURFURAL**
VERFAHREN UND SYSTEME ZUR HERSTELLUNG VON FURFURAL
PROCÉDÉS ET SYSTÈMES DE PRODUCTION DE FURFURAL

(30) Priority: 18.12.2020 EP 20215448
(43) Date of publication of application: 25.10.2023
(73) Proprietor: Shell Internationale Research Maatschappij B.V., 2596 HR The Hague (NL)
(72) Inventor: LANGE, Jean Paul Andre Marie Joseph Ghislain, 1031 HW Amsterdam (NL); RICCIARDI, Luca, 7522 NB Enschede (NL); VERBOOM, Willem, 7522 NB Enschede (NL); HÜSKENS, Jurriaan, 7522 NB Enschede (NL)
(74) Representative: Shell Legal Services IP
(86) International application number: PCT/US2021/064132
(87) International publication number: WO 2022/133271

(56) References cited:
- WO-A1-2015/066287
- WO-A1-2015/066287

## Description

### FIELD OF THE INVENTION

The present invention relates to methods and systems for production of furfural from a xylose-containing solution.

### BACKGROUND OF THE INVENTION

Furfural plays an important role in the chemical industry as a precursor of furan and derivatives of furan, including furfuryl alcohol. Also, furfural is used for the production of resins by condensation reaction of furfural with formaldehyde, phenol, acetone or urea. In addition, furfural can be used as a solvent, vulcanization enhancer, insecticide, fungicide, germicide, or in the production of such compounds, as well as for use as a potential fuel.

Furfural is an attractive compound because it can be produced from renewable resources. One potential source of renewable (non-fossil) feedstock for the production of furfural are substances selected from the group consisting of xylose, oligosaccharides comprising xylose units and polysaccharides comprising xylose units originating from cellulose-containing biomass.

Xylose is a monosaccharide also referred to as wood sugar which belongs to the group of pentoses. Oligo- and polysaccharides which comprise xylose units typically occur in plants, especially in woody parts of plants, in straw, and in the seeds or the shells of the seeds of several plants. Oligo- and polysaccharides which consist of xylose units are generally referred to as xylans. Oligo- and polysaccharides which consist of xylose units and other monosaccharide units are generally referred to as heteroxylans. Xylans and heteroxylans belong to the group of polyoses. Polyoses (earlier also referred to as hemicellulose) are polysaccharides which in plant biomass typically occur in a composite wherein said polyoses and lignin are incorporated between cellulose fibres. Dry plant biomass (water content below 15 wt.%) which comprises cellulose, polyoses and lignin is also referred to hereinabove and hereinbelow as lignocellulose.

One general process to produce furfural from xylose in biomass material is "aqueous dehydration" using batchwise or continuous acid-catalysed dehydration. This type of aqueous dehydration process provides a yield of about 30-50 mol% furfural (meaning only 30 - 50 % of the total moles of xylose is converted to furfural) (Furfural - a promising platform for lignocellulosic biofuels by J.-P. Lange, E. van der Heide, J. van Buijtenen, R.J. Price, ChemSusChem 2012, 5, 150-166). With such low yields, it degrades 50-70 mol% of the valuable xylose into undesirable by-products that foul equipment and contaminate the water stream.

Another general process which has improved yields over aqueous dehydration is "biphasic dehydration," which adds a water-insoluble solution to the aqueous dehydration to extract the furfural into an organic phase to protect it from further degradation, and optionally a salt in the aqueous phase to further assist the extraction of furfural into the water-insoluble solution (Lange et al. 2012). While biphasic dehydration can increase the yield to 60 to 70 mol%, it still degrades 30 to 40 mol% of the valuable xylose into undesirable by-products that foul equipment and build up in the solvent recycle stream.

Yet another process with improved yields over biphasic dehydration is to extract and recover xylose as a solid product from hydrolysis of biomass and subjecting the recovered xylose in a dehydration reaction (B. R. Caes, R. T. Raines, ChemSusChem 2011, 4, 353 - 356; L. Shuai, J. Luterbacher, ChemSusChem 2016, 9,133-155). Because the xylose is not in solution, the dehydration reaction can be carried out in a single phase under conditions favourable to the furfural conversion, such as using polar aprotic solvents which can provide furfural yields of 90 mol%. However, this process requires isolation of the xylose from the hydrolysate, e.g., by distilling out all the water, which can be highly energy demanding. Such isolation process to recover the xylose in solid form can further concentrate contaminants of the xylose streams in the solid xylose end product.

Although there are processes to provide improved isolation xylose from hydrolysate, such as (US10407453), these processes nonetheless, are directed to providing the xylose in dry form to allow the xylose to then be converted and/or used in the production of a C5 sugar-platform of biochemical and biofuels.

WO2015/066287 A1 discloses a method for producing furfural, comprising: enzymatically isomerizing xylose to xylulose in an aqueous solution; extracting both xylose and xylulose with an immiscible non-aqueous phase containing a water-insoluble boronic acid (such as a phenyl boronic acid or a naphthalene boronic acid) which forms diboronate esters with the two isomers and drives the isomerisation reaction towards formation of more xylulose; extracting xylulose from the immiscible non-aqueous phase with a mixture of acidic aqueous and aprotic solvents such as DMSO; heating the mixture from previous step, containing extracted xylulose, to dehydrate xylulose to furfural.

It would, therefore, be advantageous to provide a process for the production of furfural from a xylose-containing aqueous solution with a relatively higher yield without expensive isolation of xylose in dry form.

### SUMMARY OF THE INVENTION

Accordingly, the present invention provides a process for a method for producing furfural comprising:
a. providing a xylose-containing solution comprising xylose in an amount of greater than or equal to 0.5 wt.%, wherein the xylose-containing solution is an aqueous solution;
b. providing an extraction solution comprising a water-insoluble boronic acid (BA: R-B(OH)₂) and a water-insoluble solvent;
c. combining the xylose-containing solution with the extraction solution to provide a first combined solution, wherein the ratio of boronic acid to xylose in the first combined solution is greater than 1:1 molar, respectively, and wherein the first combined solution comprises a first aqueous phase and a first non-aqueous phase, said non-aqueous phase comprising at least a portion of the xylose as xylose-diboronate ester (BA₂X);
d. separating at least a portion of the first non-aqueous phase from the first combined solution;
e. providing a conversion solution comprising a water-soluble solvent and water, said conversion solution has a pH of less than or equal to 4;
f. combining at least a portion of the non-aqueous phase from (d) with the conversion solution in a ratio of conversion solution to the non-aqueous phase in a range from 0.1 to 10.0 by weight, respectively, to form a second combined solution;
g. heating the second combined solution to a temperature Tₕ at or above which the second combined solution consists essentially of a homogeneous liquid phase, wherein such heating converts at least a portion of the xylose-diboronate ester into furfural,
h. cooling down the heated second combined solution such that the cooled second combined solution comprises (i) a second aqueous phase comprising water, water-soluble solvent, and furfural and (ii) a second non-aqueous phase comprising water-insoluble solvent, water-insoluble boronic acid, and furfural; and separating at least a portion of the second non-aqueous phase from the cooled second combined solution; and
i. recovering at least a portion of the furfural in the second non-aqueous phase.

The present disclosure also provides for a method for producing furfural comprising:
a. providing a xylose-containing solution comprising xylose in an amount of greater than or equal to 0.5 wt.%, wherein the xylose-containing solution is an aqueous solution;
b. providing an extraction solution comprising a water-insoluble boronic acid (BA: R-B(OH)₂) and a water-insoluble solvent;
c. combining the xylose-containing solution with the extraction solution to provide a first combined solution, wherein the ratio of boronic acid to xylose in the first combined solution is greater than 1:1 molar, respectively, and wherein the first combined solution comprises a first aqueous phase and a first non-aqueous phase, said non-aqueous phase comprising at least a portion of the xylose as xylose-diboronate ester (BA₂X);
d. separating at least a portion of the first non-aqueous phase from the first combined solution;
e. providing a conversion solution comprising a water-soluble solvent and water, said conversion solution has a pH of less than or equal to 4;
f. combining at least a portion of the non-aqueous phase from (d) with the conversion solution in a ratio of conversion solution to the non-aqueous phase in a range from 0.1 to 10.0 by weight, respectively, to form a second combined solution;
g. heating the second combined solution to a temperature of greater than or equal to 100°C to convert at least a portion of the xylose-diboronate ester into furfural, wherein the heated second combined solution comprises a second aqueous phase comprising water, at least a portion of the water-soluble solvent from the conversion solution, and furfural and (ii) a second non-aqueous phase comprising at least a portion of water-insoluble solvent and water-insoluble boronic acid from the extraction solution, and furfural,
h. separating at least a portion of the second non-aqueous phase from the cooled second combined solution; and
i. recovering at least a portion of the furfural from the non-aqueous phase.

Optionally, step (i) comprises providing at least a portion of the second non-aqueous phase from (h) to a distillation process to recover an overhead product comprising furfural and a bottom product comprising water-insoluble solvent and water-insoluble boronic acid. Optionally, the method further comprises providing at least a portion of the bottom product for use as part of the extraction solution. Optionally, at least a portion of the first aqueous phase in (c) comprises water-soluble solvent, water-insoluble solvent, and water-insoluble boronic acid, said method further comprising: separating at least a portion of the first aqueous phase in (c) from the first combined solution; and further processing at least a portion of the separated first aqueous phase to recover at least one of water-soluble solvent, water-insoluble solvent, and water-insoluble boronic acid.

Optionally, the method further comprises performing at least a portion of steps (c) and (d) in a liquid-liquid extraction unit in counter-current operation, wherein the xylose-containing solution is provided at a higher temperature than the temperature of the extraction solution.

Optionally, the method further comprises providing at least a portion of the second aqueous phase from (h) to a distillation process to recover an overhead product comprising water and furfural and an acidic bottom product comprising water and water-soluble solvent, wherein the bottom product has a pH of less than 7. Optionally, the method further comprises providing at least a portion of the acidic bottom product for use as part of the conversion solution.

Optionally, the method further comprises providing at least a portion of the second aqueous phase from (h) to a solvent-extraction process to recover furfural, wherein at least a portion of solvent used in the extraction process comprises the distillation bottom product comprising water-insoluble solvent and water-insoluble boronic acid.

Optionally, the xylose-containing solution is a hydrolysate. Optionally, the water-insoluble boronic acid has up to 5 wt.% solubility in water at 20°C. Optionally, the water-insoluble boronic acid is selected from the group consisting of phenylboronic acid, 4-biphenylboronic acid, 4-butylphenyl boronic acid, 4-tert-Butylphenyl boronic acid, 4-ethylphenyl boronic acid, 2-naphthylboronic acid, naphthalene-1-boronic acid, o-tolylboronic acid, m-tolylboronic acid, (2-methylpropyl) boronic acid, butylboronic acid, octylboronic acid, phenethyl boronic acid, cyclohexyl boronic acid, and any combination thereof. Optionally, the water-insoluble solvent has up to 5 wt.% solubility in water at 20°C. Optionally, the water-insoluble solvent is selected from the group consisting of benzoic acid, cresol (m), di-isopropyl ether, terephthalic acid, diethylene glycol diethyl ether, anisole, salicylic acid, 2,6 xylenol, 4Et-phenol, toluene, benzofuran, ethylbenzene, octanoic acid, 1-methylnaphtalene, nitrobenzene, guaiacol, heptane, 1-octanol, and methylisobutyl ketones, an any combination thereof. Optionally, at least one of the water-insoluble boronic acid and water-insoluble solvent has a boiling point higher than that of furfural, preferably at least 5°C higher. Optionally, the water-soluble solvent has a logP (octanol-water partition co-efficient) in a range from (-3) to 0. Optionally, the water-soluble solvent is selected from the group consisting of dimethyl sulfoxide, diglyme, sulfolane, gamma butyrolactone, succinic acid, nMe-acetamide, dioxane, nMe-pyrrolidone, gamma valerolactone, acetone, Acetic acid, and any combination thereof. Optionally, the water-soluble solvent has a boiling point higher than that of water, preferably at least 5 °C higher.

### BRIEF DESCRIPTION OF THE DRAWINGS

Figure 1 illustrates one embodiment of the processes to produce furfural according to certain aspects disclosed herein.
Figure 2 illustrates another embodiment of the process to produce furfural according to certain aspects disclosed herein.
Figure 3 illustrates one embodiment of the systems to produce furfural according to certain aspects disclosed herein.
Figure 4 illustrates another embodiment of the systems to produce furfural according to certain aspects disclosed herein.
Figure 5 illustrates one embodiment of the processes to recover xylose according to certain aspects disclosed herein.

### DETAILED DESCRIPTION OF THE INVENTION

The present invention will now be described in detail with reference to embodiments thereof as illustrated in the accompanying drawings. References to "one embodiment", "an embodiment" "an example embodiment", etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. Other suitable modifications and adaptations of the variety of conditions and parameters normally encountered in the field, and which would be apparent to those skilled in the art, are within the scope of the invention.

Although the description herein provides numerous specific details that are set forth for a thorough understanding of illustrative embodiments, it will be apparent to one skilled in the art that embodiments may be practiced without some or all of these specific details. In other instances, well known process steps and/or structures have not been described in detail in order to not unnecessarily obscure the present invention. The features and advantages of embodiments may be better understood with reference to the drawings and discussions that follow.

In addition, when like elements are used in one or more figures, identical reference characters will be used in each figure, and a detailed description of the element will be provided only at its first occurrence. Some features or components of the systems or processes described herein may be omitted in certain depicted configurations in the interest of clarity. Moreover, certain features such as, but not limited to pumps, valves, gas bleeds, gas inlets, fluid inlets, fluid outlets and the like have not necessarily been depicted in the figures, but their presence and function will be understood by one having ordinary skill in the art. Similarly, the depiction of some of such features in the figures does not indicate that all of them are depicted.

The present inventors have surprisingly found that xylose in an aqueous solution can be extracted as xylose-diboronate ester (BA₂X) into the non-aqueous phase of an extraction solution comprising a water-insoluble solvent and a water-insoluble boronic acid, and the non-aqueous phase can be separated for conversion or dehydration of the xylose-diboronate ester (BA₂X) into furfural with an acidic conversion solution comprising water and a water-soluble solvent. The furfural can then be recovered using any suitable methods. Optionally, instead of or in addition to making furfural, the xylose-diboronate ester (BA₂X) in the separated non-aqueous phase can be back-extracted into xylose to recover the xylose away from impurities or other undesired compounds that may be present in the initial xylose solution.

As used herein, "aqueous solution" has its ordinary meaning, which is a solution in which a solute is dissolved in a solvent, and the solvent is water. "Water-insoluble" also has its ordinary meaning, which describes the low solubility of a substance in water. Low solubility means preferably up to 5 wt.% solubility at 20 °C, including up to 2 wt.% solubility, up to 1 wt.% solubility, up to 0.5 wt.% solubility, or up to 0.1 wt.% solubility. Alternatively, "water-insoluble" as used herein describes a substance with an octanol-water partition coefficient LogP (also called LogKow) of at least 1, including at least 2, or at least 3. "Water-soluble" as used herein describes a substance with a LogP in a range from (-3) to 0, preferably from (-2.5) to (-0.5), more preferably (-2.0) to (-1.0). "Aqueous phase" has its ordinary meaning, which describes a liquid phase in which the concentration of water is greater than the concentration of water-insoluble liquid component(s). "Non-aqueous" has its ordinary meaning, which describes a liquid phase in which the concentration of water-insoluble liquid component(s) is greater than the concentration of water.

The present disclosure provides for a process for producing furfural comprising:
a. providing a xylose-containing solution comprising xylose in an amount of greater than or equal to 0.5 wt.%, wherein the xylose-containing solution is an aqueous solution;
b. providing an extraction solution comprising a water-insoluble boronic acid (BA: R-B(OH)₂) and a water-insoluble solvent;
c. combining the xylose-containing solution with the extraction solution to provide a first combined solution, wherein the ratio of boronic acid to xylose in the first combined solution is greater than 1:1 molar, respectively, and wherein the first combined solution comprises a first aqueous phase and a first non-aqueous phase, said non-aqueous phase comprising at least a portion of the xylose as xylose-diboronate ester (BA₂X);
d. separating at least a portion of the first non-aqueous phase from the first combined solution;
e. providing a conversion solution comprising a water-soluble solvent and water, said conversion solution has a pH of less than or equal to 4;
f. combining at least a portion of the non-aqueous phase from (d) with the conversion solution in a ratio of conversion solution to the non-aqueous phase in a range from 0.1 to 10.0 by weight, respectively, to form a second combined solution;
g. heating the second combined solution to a temperature Tₕ at or above which the second combined solution consists essentially of a homogeneous liquid phase, wherein such heating converts at least a portion of the xylose-diboronate ester into furfural,
h. cooling down the heated second combined solution such that the cooled second combined solution comprises (i) a second aqueous phase comprising water, water-soluble solvent, and furfural and (ii) a second non-aqueous phase comprising water-insoluble solvent, water-insoluble boronic acid, and furfural; and separating at least a portion of the second non-aqueous phase from the cooled second combined solution; and
i. recovering at least a portion of the furfural in the second non-aqueous phase.

In addition, the present disclosure also provides for a method for producing furfural comprising:
a. providing a xylose-containing solution comprising xylose in an amount of greater than or equal to 0.5 wt.%, wherein the xylose-containing solution is an aqueous solution;
b. providing an extraction solution comprising a water-insoluble boronic acid (BA: R-B(OH)₂) and a water-insoluble solvent;
c. combining the xylose-containing solution with the extraction solution to provide a first combined solution, wherein the ratio of boronic acid to xylose in the first combined solution is greater than 1:1 molar, respectively, and wherein the first combined solution comprises a first aqueous phase and a first non-aqueous phase, said non-aqueous phase comprising at least a portion of the xylose as xylose-diboronate ester (BA₂X);
d. separating at least a portion of the first non-aqueous phase from the first combined solution;
e. providing a conversion solution comprising a water-soluble solvent and water, said conversion solution has a pH of less than or equal to 4;
f. combining at least a portion of the non-aqueous phase from (d) with the conversion solution in a ratio of conversion solution to the non-aqueous phase in a range from 0.1 to 10.0 by weight, respectively, to form a second combined solution;
g. heating the second combined solution to a temperature of greater than or equal to 100°C to convert at least a portion of the xylose-diboronate ester into furfural, wherein the heated second combined solution comprises a second aqueous phase comprising water, at least a portion of the water-soluble solvent from the conversion solution, and furfural and (ii) a second non-aqueous phase comprising at least a portion of water-insoluble solvent and water-insoluble boronic acid from the extraction solution, and furfural,
h. separating at least a portion of the second non-aqueous phase from the cooled second combined solution; and
i. recovering at least a portion of the furfural from the non-aqueous phase.

### Xylose-containing solution

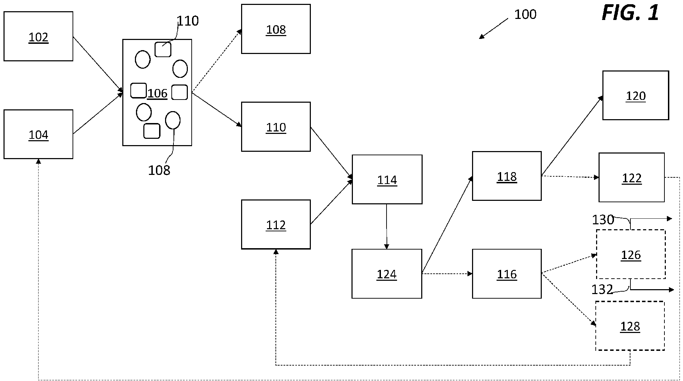

Referring to FIG. 1, the process comprises providing a xylose-containing aqueous solution 102 comprising xylose in an amount of greater than or equal to 0.5 wt.%, including preferably greater than or equal to 1.0 wt.%, more preferably greater than or equal to 2.0 wt.%, or most preferably greater than or equal to 3.0 wt.%.

The process described herein can be suitable for use with a xylose-containing aqueous solution with any pH, from 1 to 14. That is, the process described herein can be used with xylose-containing aqueous solution 102 that is acidic with a pH in a range from 1 to 6, xylose-containing aqueous solution 102 that is basic with a pH in a range of 8 to 14, or xylose-containing aqueous solution 102 that is neutral with a pH from greater than 6 to less than 8.

While any xylose-containing solution as described herein may be provided for use in the process, a suitable xylose-containing solution 102 can include one that is derived from a pre-treatment step in which a cellulosic biomass is hydrolysed by methods known by one of ordinary skill in the art, including hot water at neutral pH (e.g. steam explosion), hot water at acidic pH e.g. by addition of organic or inorganic acids (e.g. dilute acid and reversible-acid pre-treatment), or hot water at basic pH e.g. by addition of organic or inorganic base (e.g. kraft pulping), as described e.g. by Steinbach, Kruse, Sauer, Biomass Conv. Bioref. (2017) 7:247-274, as well as those methods that employ ionic liquids.

In a preferred embodiment the term "cellulosic biomass" refers to biomass comprising a) cellulose as well as b) one or more substances selected from the group consisting of polyoses and other sources of xylose units. For example, lignocellulose is cellulosic biomass that can serve as a source of xylose units. Suitable cellulosic biomass, particularly lignocellulose, includes any material and/or agricultural biomass having a lignocellulose (or hemicellulose) concentration of at least 10%, at least 15%, at least 20%, at least 25%, at least 30%, or at least 50%. Exemplary lignocellulosic biomasses that can be used in this regard include, but are not limited to: corn cobs, crop residues such as corn husks, corn stover, grasses, wheat, wheat straw, barley straw, hay, rice straw, switchgrass, waste paper, sugar cane bagasse, sorghum, components obtained from milling of grains, trees, branches, roots, leaves, wood chips, sawdust, shrubs and bushes, vegetables, fruits, flowers and animal manure, soy hulls from soybean processing, rice hulls from rice milling, corn fibre from wet milling or dry milling, bagasse from sugarcane processing, pulp from sugar beets processing, distillers grains, and the like.

Suitably, the pretreatment step as described in WO2016025678 and WO2016025679 can be used to hydrolyse cellulosic biomass to produce a xylose-containing solution that may be used in the process described herein. As noted above, such product of hydrolysis may be referred to as a hydrolysate, which comprises xylose in an amount of at least 0.5 wt.%, including preferably at least 1.0 wt.%, at least 2.0 wt.%, or most preferably at least 3.0 wt.%.

Preferably, xylose-containing aqueous solution 102 may be a product of an acidic pre-treatment based on diluted H2SO4 or concentrated HESA as acid, as described in U.S. Patent No. 9290821.

Xylose-containing solution 102 is an aqueous solution, which means it is a solution in which the solvent is liquid water.

### Extraction solution

Referring to FIG. 1, the process further comprises providing an extraction solution 104 comprising an organic or water-insoluble boronic acid (BA: R-B(OH)2) and a water-insoluble solvent.

A suitable water-insoluble boronic acid is one with low water solubility, preferably up to 5 wt.% solubility (meaning the selected water-insoluble boronic acid is soluble up to 5 wt.% in water at 20°C), including up to 2 wt.% solubility, up to 1 wt.% solubility, up to 0.5 wt.% solubility, or up to 0.1 wt.% solubility. Alternatively, a suitable water-insoluble boronic acid is one with an octanol-water partition coefficient LogP (also called LogKow) of at least 1, including at least 2, or at least 3.

In a preferred embodiment, a suitable water-insoluble boronic acid has an atmospheric boiling point that is higher than the atmospheric boiling point (Tb) of furfural, which is 162°C, to allow for use of distillation as an option for recovery of furfural as an overhead product. Preferably, the suitable water-insoluble boronic acid has an atmospheric boiling point that is at least 2 °C higher (i.e., atmospheric boiling point of at least 164C), more preferably at least 5 °C (i.e., atmospheric boiling point of at least 167C).

For instance, one exemplary suitable water-insoluble boronic acid is phenyl boronic acid, which has a water solubility of 1 wt.% at 20°C and a logP of 1.59 and an atmospheric boiling point Tb of 265°C. Other substituted phenyl boronate compounds are also suitable, such as alkyl phenyl boronate, naphthanyl boronate and substituted naphthanyl boronate, and any combination thereof. For example, one suitable example is 2-napthyl boronic acid, which has a water solubility of 0.03 wt.% at 20°C and a logP of 2.82 and a Tb of 382°C. Another example is octylboronic acid which is reported as water-insoluble and has a logP of 3.56 and a Tb of 262°C.

Table 1 below shows examples of suitable water-insoluble boronic acid that can be used in extraction solution 104, along with their solubility description and/or LogP, and atmospheric boiling point (Tb).

**Table 1**

| **Name** | **Water solubility g/L (20°C)** | **LogP*** | **Tb*** |
|---|---|---|---|
| | | | **°C** |
| phenylboronic acid | 10 | 1.59 | 265 |
| 4-biphenylboronic acid | Insoluble | 3.35 | |
| 4-butylphenyl boronic acid | Insoluble | 3.64 | |
| 4-*tert*-Butylphenyl boronic acid | Insoluble | 3.28 | |
| 4-ethylphenyl boronic acid | N.A. | 2.58 | |
| 2-naphthylboronic acid | 0.3 | 2.82 | 382 |
| naphthalene-1-boronic acid | 0.3 | | |
| *o*-tolylboronic acid | Slightly soluble | 2.05 | 283 |
| *m*-tolvlboronic acid | 25 | 2.05 | 290 |
| (2-methylpropyl) boronic acid | 33 | 0.6 | 180 |
| butvlboronic acid | 25 | 1.43 | 189 |
| octvlboronic acid | Insoluble | 3.56 | 262 |
| Phenethyl boronic acid | 1.8 | 1.20 | |
| Cvclohexvl boronic acid | 25 | 1.90 | 253 |

One or more (such as two or more) suitable water-insoluble boronic acid as described here can be used in extraction solution 104 as described herein based on design choices by one of ordinary skill in the art. While certain descriptions may refer to "a water-insoluble boronic acid," "the water-insoluble boronic acid," or "the boronic acid," it is understood that such reference can include more than one (such as two or more) water-insoluble boronic acids, as applicable.

Referring to FIG. 1, extraction solution 104 further comprises a water-insoluble solvent. A suitable water-insoluble solvent is one with low water solubility to lower the likelihood of dissolution in water, preferably up to 5 wt.% solubility (meaning the selected water-insoluble solvent is soluble up to 5 wt.% of in water at 20°C), including up to 2 wt.% solubility, up to 1 wt.% solubility, up to 0.5 wt.% solubility, or up to 0.1 wt.% solubility. Alternatively, a suitable water-insoluble solvent is one with an octanol-water partition coefficient LogP (also called LogKow) of at least 1, including at least 2, or at least 3 and up to 7, up to 6, or up to 5, preferably from 1 to 7, more preferably from 2 to 6, and most preferably from 3 to 5.

In a preferred embodiment, a suitable water-insoluble solvent has an atmospheric boiling point that is higher than the atmospheric boiling point of furfural, which is 162°C, to allow for use of distillation as an option for recovery of furfural as an overhead product. Preferably, the suitable water-insoluble boronic acid has an atmospheric boiling point that is at least 2 °C higher (i.e., atmospheric boiling point of at least 164°C), more preferably at least 5 °C (i.e., atmospheric boiling point of at least 167°C).

In a preferred embodiment, the water-insoluble solvent has a good affinity for the xylose-diboronate ester and a good affinity for furfural. By affinity we mean a high partition coefficient of the BA₂X or furfural between extraction solution 104 and water in first combined solution 106 (described further below), including such partition coefficient of at least 0.1, preferably at least 0.5, preferably at least 1.0, and most preferably at least 2.

Examples of suitable water-insoluble solvent that have good affinity for the xylose-diboronate ester and a good affinity for furfural include aromatic hydrocarbons, preferably toluene and most preferably methyl naphthalene or aromatic mixtures rich in alkylbenzene or alkyl-naphthalene components. Water-insoluble solvent also include aromatic components that carry heteroatoms such as nitrobenzene, anisole, guaiacol, cresols, as well as aliphatic components free of heteroatoms (e.g., heptane and other alkanes) or containing heteroatoms (e.g., 1-octanol, methylisobutyl ketones)

Table 2 below shows examples of suitable water-insoluble solvents that can be used in extraction solution 104, along with their solubility (LogP) and atmospheric boiling point (Tb).

**Table 2**

| | Water solubility g/L (20 °C) | LogP | Tb °C |
|---|---|---|---|
| guaiacol | 18.7 (25°C) | 0.97 | 205 |
| methylisobutyl ketone | 19 | 1.25 | 117 |
| nitrobenzene | 2.1 (25°C) | 1.85 | 210 |
| Benzoic acid | 3.4 | 1.87 | 249 |
| Cresol (m) | 23 | 1.94 | 203 |
| Diisopropyl ether | 1.7 | 2.03 | 68 |
| terephthalic acid | 0.015 | 2.00 | 392 |
| anisole | 1.5 | 2.11 | 153 |
| Salicylic acid | 2.0 | 2.26 | high |
| 2,6 xylenol | 6.2 | 2.40 | 201 |
| 4Et-phenol | 6.1 | 2.47 | 218 |
| Toluene | 0.52 (25°C) | 2.50 | 111 |
| Benzofuran | insoluble | 2.67 | 174 |
| Ethylbenzene | 0.15 | 2.96 | 136 |
| 1-octanol | 0.3 | 3.10 | 195 |
| octanoic acid | 0.9 | 3.32 | 237 |
| 1-methylnaphtalene | 0.026 | 3.63 | 241 |
| heptane | 0.003 | 4.47 | 98 |

One or more (such as two or more) suitable water-insoluble solvents as described here can be used in extraction solution 104 as described herein based on design choices by one of ordinary skill in the art. While certain descriptions may refer to "a water-insoluble solvent" or "the water-insoluble solvent," it is understood that such reference can include more than one (such as two or more) water-insoluble solvents, as applicable.

Suitably, extraction solution 104 can comprise at least 1 wt.%, including at least 5 wt.%, at least 10 wt.%, or at least 20 wt.% of the water-insoluble boronic acid and up to 99 wt.%, including up to 95 wt.%, up to 90 wt.%, or up to 80 wt.% of the water-insoluble solvent.

### First combined solution

Referring to FIG. 1, the process further comprises combining an amount of xylose-containing solution 102 with an amount of extraction solution 104 to provide first combined solution 106, wherein the ratio of boronic acid to xylose in first combined solution 106 is at least 1:1 molar, respectively, preferably at least 1.5:1 and most preferably at least 2:1. It is understood that one of ordinary skill would be capable of (i) determining the amount of xylose in xylose-containing solution 102 (if such amount is unknown) using methods such as high-pressure liquid chromatography (HPLC), (ii) preparing extraction solution 104 with a suitable amount of water-insoluble boronic acid and water-insoluble solvent, in accordance with the description provided herein, and (iii) determining the amount of such extraction solution 104 to be added to a particular amount of xylose-containing solution 102 so that the ratio of boronic acid to xylose in first combined solution 106 is at least 1:1 molar.

When xylose-containing solution 102 is combined with extraction solution 104 to form first combined solution 106, at least a portion of the xylose from solution 102 comes into contact with at least a portion of organic boronic acid from solution 104. This contact allows the xylose to be converted to xylose monoboronate and subsequently xylose-diboronate ester, which has low solubility in water, so it has a greater affinity toward the water-insoluble solvent of solution 104 that is in solution 106. As more xylose is converted to xylose-diboronate esters, non-aqueous phase 110 comprising (i) boronic acid and water-insoluble solvent from extraction solution 104 and (ii) xylose-diboronate esters begins to form. Correspondingly, aqueous phase 108 with less xylose than xylose-containing solution 102 begins to form as well.

After an amount of time, which can be determined or selected by one of ordinary skill to achieve certain desired objectives, first combined solution 106 comprises aqueous phase 108 and a non-aqueous phase 110, said non-aqueous phase comprising at least a portion of the xylose from xylose-containing solution 102 as xylose-diboronate ester (BA₂X). Preferably, non-aqueous phase 110 comprises at least 20 mol% of the xylose from xylose-containing solution 102 as xylose-diboronate ester, more preferably at least 50 mol%, including at least 70 mol% or at least 90 mol%.

Preferably, first combined solution 106 is mixed using suitable methods, such as mixing, stirring, static mixer, turbulent flow, jet loop, etc to allow xylose and organic boronic acids molecules to interact, thereby improving the yield of xylose-diboronate-ester that forms. Examples of suitable methods for mixing can additionally or alternatively include internal components of the separation or extraction units noted above. Suitably, the mixing may be performed for at least 30 minutes, preferably at least 60 minutes, and most preferably at least 90 minutes.

Referring to FIG. 1, the process further comprises separating at least a portion of non-aqueous phase 110 from first combined solution 106. While FIG. 1 shows aqueous phase 108 also being separated, this is optional and is included in FIG. 1 to illustrate the two phases (108 and 110) being described. While the steps of forming first combined solution 106 and separating non-aqueous phase 110 can be performed separately, they can suitably be carried out at least partially (and/or fully) together via a liquid-liquid extraction or separation process as further described below.

After at least a portion of the xylose in first combined solution 106 has been allowed to react to form xylose-diboronate esters and first combined solution 106 comprises aqueous phase 108 and non-aqueous phase 110, the phases 108 and 110 can be separated using suitable methods, such as liquid-liquid extraction or separation methods, suitably in co-current flow and preferably counter-current flow. It is understood by one of ordinary skill that aqueous phase 108 can contain an amount of water-insoluble boronic acid and water-insoluble solvent from extraction solution 104 but the concentration of water in aqueous phase 108 is higher than the concentration of water-insoluble components from extraction solution 104. Similarly, it is understood that non-aqueous phase 110 can contain an amount of water but the concentration of water-insoluble boronic acid and water-insoluble solvent in non-aqueous phase 110 is higher than the concentration of water.

Such extraction or separation methods can be performed using a series of mixers-decanters but can also be performed in a unit or series of units that integrates mixing and decanting and is preferably operated in counter current flow. Such unit can optionally contain internal components to facilitate the mixing and decanting, including stationary components (trays, random or structured packings) or agitators (e.g., rotating or oscillating disks).

Referring to FIG. 1, forming of first combined solution 106 and separating of non-aqueous phase 110 can be performed at ambient temperature (at least 20 °C), and optionally either or both can be performed at elevated temperature, such as at least 30 °C, at least 50 °C, or at least 90 °C. One exemplary factor to consider in selecting an elevated temperature is that higher temperatures will allow shorter extraction times to facilitate the rate of reaction of xylose with boronic acid, and correspondingly, the conversion from xylose to xylose-diboronate ester in first combined solution 106. There are other factors for consideration known to one of ordinary skill in selecting conditions to perform step 106 and/or step 108, such as energy requirements.

In one embodiment, forming of first combined solution 106 and separating of non-aqueous phase 110 can be performed isothermally (e.g., temperature is the same for both steps). Additionally, or alternatively, they can be performed under a temperature gradient. One suitable way of providing a temperature gradient is to provide xylose-containing solution 102 at a higher temperature than extraction solution 104. For instance, the temperature of xylose-containing solution 102 can be at least 5 °C higher than the temperature of extraction solution 104, preferably at least 10 °C higher, more preferably at least 15 °C higher, and most preferably at least 20 °C higher. Referring to FIG. 1, the at least a portion of non-aqueous phase 110, preferably at least 20% of non-aqueous phase 110, is separated from first combined solution 106 using one or more suitable separation methods as described above. More preferably, at least 50%, including at least 70%, or at least 90% of non-aqueous phase 110 is separated from first combined solution 106. Preferably, such separation is achieved using at least liquid-liquid separation via gravity whereby phases 108 and 110 are retained in a separator at least an amount of time that allows phases 108 and 110 to separate by the differences in density of phases 108 and 110, with aqueous phase 108 typically being above non-aqueous phase 110. It is known to one of ordinary skill that such separation via liquid-liquid separation can also be facilitated by enhanced gravity using, for example, hydrocyclone and centrifugation devices. It is understood that one of ordinary skill would be capable of selecting the suitable separator specifications (such as size, configuration, including whether and which internal components to include to facilitate mixing and decanting, arrangement, including whether to use dedicated units (e.g., mixer-decanter) and/or integration with the extraction unit, and whether and which enhanced gravity devices to include) and suitable amount of time to achieve an equilibrium condition between phases 108 and 110 at the temperature and pressure of separation of first combined solution 106 to allow for separation and/or removal of at least a portion of non-aqueous phase 110 from first combined solution 106, which effectively removes non-aqueous phase 110 from aqueous phase 108. As noted above, the xylose-diboronate ester has lower solubility in water, which means non-aqueous phase 110 comprises a greater portion of the xylose-diboronate ester than aqueous phase 108. Separation of non-aqueous phase 110 also separates or removes a greater portion of the xylose-diboronate ester from first combined solution 106.

### Conversion solution

Referring to FIG. 1, the process further comprises combining at least a portion of the separated portion of non-aqueous phase 110 with conversion solution 112 to form second combined solution 114, wherein conversion solution 112 comprises a water-soluble solvent and water and has a pH of less than or equal to 4. Preferably, at least 20% of the separated non-aqueous phase 110 is combined with conversion solution 112, more preferably at least 50%, including at least 70%, at least 90%, or all of the separated non-aqueous phase 110 is combined with conversion solution 112. The ratio of conversion solution 112 to non-aqueous phase 110 in second combined solution 114 is in a range from 0.1 to 10.0, preferably from 0.3 to 5.0, and more preferably from 0.5 to 2.0, by weight, respectively. Notably, second combined solution 114 comprises four major components: water-insoluble boronic acid and water-insoluble solvent from extraction solution 104, and water-soluble solvent and water from conversion solution 112.

Conversion solution 112 comprises water and a water-soluble solvent. Suitably, the amount of water-soluble solvent in conversion solution is in a range of 1 wt.% to 95 wt.%, preferably from 10 wt.% to 90 wt.%, more preferably from 20 wt.% to 80 wt.%, and most preferably from 30 wt.% to 70 wt.%. Suitably, the water-soluble solvent has a LogP (octanol-water partition co-efficient) in a range from (-3) to 0, preferably from (-2.5) to (-0.5), more preferably (-2.0) to (-1.0), which facilitate solubilizing of the water-insoluble solvent in water.

One or more water-soluble solvents may be used in aqueous conversation solution 112 as described herein based on design choices by one of ordinary skill in the art. While certain descriptions may refer to "a water-soluble solvent" or "the water-soluble solvent," it is understood that such reference can include more than one (such as two or more) water-insoluble solvents, as applicable. Examples of suitable water-soluble solvent include dioxane, GVL (gamma-valerolactone), dimethyl sulfoxide (DMSO), and sulfolane, and any combination thereof.

Table 3 below shows examples of suitable water-soluble solvents that can be used in conversion solution 112, along with their solubility (LogP).

**Table 3**

| | LogP |
|---|---|
| DMSO | -1.35 |
| Diglyme | -0.8 |
| sulfolane | -0.77 |
| gamma butyrolactone | -0.76 |
| succinic acid | -0.59 |
| nMe-acetamide | -0.75 |
| dioxane | -0.42 |
| nMe-pyrrolidone | -0.4 |
| gamma valerolactone | -0.27 |
| acetone | -0.24 |
| Acetic acid | -0.13 |

Conversion solution 112 has a pH of less than or equal to 4, preferably less than or equal to 2. Any suitable acid, preferably inorganic acid (or combination of suitable acids) can be used to lower the pH of conversion solution 112 to the desired acidic pH. Examples of suitable acids include: H₂SO₄, HCl, H₃PO₄, methane sulfonic acid, formic acid, acetic acid, trifluoro acetic acid, trichloro acetic acid, and any combinations thereof.

### Second combined solution: dehydration reaction

Referring to FIG. 1, the process further comprises heating the second combined solution to a temperature Th at or above which the second combined solution consists essentially of a homogeneous liquid phase, wherein such heating converts at least a portion of the xylose-diboronate ester into furfural. At least an aqueous phase and a non-aqueous phase of the second combined solution become a homogeneous liquid phase.

Referring to FIG. 1, when the xylose-diboronate esters from non-aqueous phase 110 in second combined solution 114 come into contact and react with water molecules generally from conversion solution 112, the xylose-diboronate esters are converted to furfural via a dehydration reaction.

Preferably at least 20 mol% of the xylose-diboronate ester in second combined solution 114 is converted into furfural, more preferably at least 50 mol%, including at least 70 mol% or at least 90 mol%, of the xylose-diboronate ester is converted into furfural. By heating second combined solution 114 to temperature Th where it consists essentially of a homogenous liquid phase, at least 80 mol% of the xylose-diboronate ester in second combined solution 114 is converted into furfural, preferably at least 90 mol%.

Suitably, second combined solution 114 may be heated at temperature Th for at least 1 minute, preferably at least 10 minutes, more preferably at least 30 minutes, most preferably at least 60 minutes, and preferably up to 10 hours, more preferably up to 5 hours, and most preferably up to 3 hours. For instance, the amount of time second combined solution 114 is preferably heated at temperature Th is in a range from 10 minutes to 10 hours, more preferably from 30 minutes to 5 hours, and most preferably from 60 minutes to 3 hours.

This Th temperature depends at least on (i) the properties and concentration of the water-soluble solvent in conversion solution 112 and the water-insoluble solvent in non-aqueous phase 110 and (ii) the properties and concentration of the boronic acid that is bound to xylose and/or free from xylose in second combined solution 114. As such, it is within the knowledge of one of ordinary skills to determine the Th for the particular conditions of a certain process as designed by such one of ordinary skills in accordance with the disclosures herein, such as composition of second combined solution 114 which depends on composition of upstream elements. Preferably, Th is in a range from at least 160 °C, more preferably at least 180 °C, and most preferably at least 220 degrees.

Referring to FIG. 1, second combined solution 114 is preferably mixed at least a portion of the time it is heated at temperature Th, said mixing is performed using suitable methods such as at least those described herein. Such mixing can further facilitate contact between water molecules and the xylose-diboronate esters for conversion to furfural. Suitable mixing methods include mixing, stirring, static mixer, turbulent flow, jet loop, etc. Suitably, the mixing may be performed for at least 30 minutes, preferably at least 60 minutes, and most preferably at least 90 minutes.

Referring to FIG. 1, process 100 further comprises cooling down heated second combined solution 114 to a temperature at or below which the homogenous liquid phase separates into an aqueous phase (not shown) and a non-aqueous phase (not shown) in cooled second combined solution 124. Such temperature may be referred to as Ts. In particular, cooled second combined solution 124 comprises an aqueous phase comprising water, water-soluble solvent, and furfural and a non-aqueous phase comprising boronic acid, water-soluble solvent, and furfural. Preferably, the temperature Ts is at least 5 °C below Th, more preferably at least 20 °C below Th, including at least 80 °C, at least 100 °C, or at least 120 °C below Th.

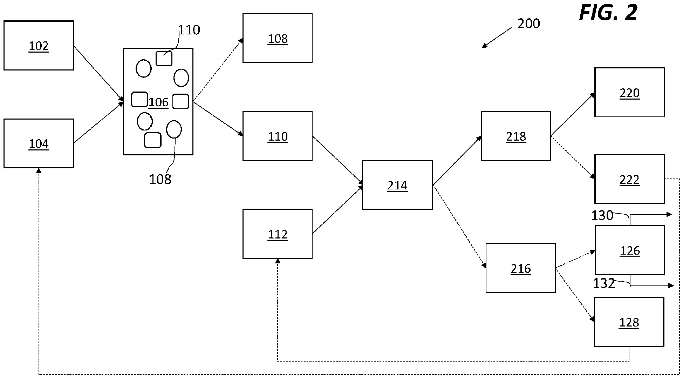

While process 100, particularly heating of second combined solution 114 to temperature Th, may be preferred to provide an optimal rate of conversion of xylose-diboronate ester to furfural, such heating is not necessary to produce furfural in accordance with the processes and systems disclosed herein. FIG. 2 depicts process 200 which involves heating of second combined solution to temperature below temperature Th, meaning the aqueous and non-aqueous phases of such heated second combined solution does not become a homogenous phase. As noted above, when like elements in FIG. 1 are also in FIG. 2 or other figures, identical reference characters will be used in each figure. Detailed description of these elements, whether disclosed below or above, apply equally for each figure, and will not be repeated verbatim for readability.

Referring to FIG. 2, process 200 comprises heating second combined solution 214 to a temperature from 100°C to convert at least a portion of the xylose-diboronate ester into furfural, wherein heated second solution 214 comprises an aqueous phase (not shown) comprising water, water-soluble solvent, and furfural and a non-aqueous phase (not shown) comprising boronic acid, water-soluble solvent, and furfural.

Suitably, second combined solution 214 may be heated for at least 1 minute, preferably at least 10 minutes, more preferably at least 30 minutes, most preferably at least 60 minutes, and preferably up to 10 hours, more preferably up to 5 hours, and most preferably up to 3 hours. For instance, the amount of time second combined solution 214 is preferably heated at a temperature of at least 100 °C is in a range from 10 minutes to 10 hours, more preferably from 30 minutes to 5 hours, and most preferably from 60 minutes to 3 hours.

Preferably at least 20 mol% of the xylose-diboronate ester in second combined solution 214 is converted into furfural, more preferably at least 50 mol%, and most preferably least 70 mol% of the xylose-diboronate ester is converted into furfural.

Referring to FIG. 2, second combined solution 214 is preferably mixed at least a portion of the time it is heated, said mixing is performed using suitable methods such as at least those described herein. Such mixing can further facilitate contact between water molecules and the xylose-diboronate esters for conversion to furfural. Suitable mixing methods include mixing, stirring, static mixer, turbulent flow, jet loop, etc. Suitably, the mixing may be performed for at least 30 minutes, preferably at least 60 minutes, and most preferably at least 90 minutes.

It is understood by one of ordinary skill that aqueous phases 116 and 216 can contain an amount of water-insoluble boronic acid and water-insoluble solvent, but the concentrations of water and water-soluble solvent are higher than those in non-aqueous phase 110. Similarly, it is understood that non-aqueous phases 118 and 218 can contain an amount of water and water-soluble solvent but the concentrations of water-insoluble solvent and water-insoluble boronic acid are higher than those in conversion solution 112.

Non-aqueous phase 118 or 218 comprises at least a portion of the produced furfural in second combined solution 114/124 or 214, respectively, including at least 10 wt.%, preferably at least 20 wt.%, more preferably at least 30 wt.%, and most preferably at least 40 wt.%. At least a portion of the furfural in non-aqueous phase 118 or 218 can be recovered by suitable methods, such as distillation where the furfural is part of an overhead product.

Referring to FIGS. 1 and 2, processes 100 and 200 enable conversion of at least a portion of the xylose-diboronate ester in second combined solution 114/124 and 214, respectively, into furfural. Preferably at least 20% of the xylose-diboronate ester in second combined solution 114 or 214 is converted to furfural, more preferably at least 50 mol%, including at least 70 mol% and at least 90 mol%.

Referring to FIGS. 1 and 2, process 100 and 200 further comprise recovering at least a portion of the produced furfural using any suitable methods known to one of ordinary skills. For instance, referring to FIG. 1, at least non-aqueous phase 118 and aqueous phase 116 may be separated from cooled second combined solution 124, and referring to FIG. 2, at least non-aqueous phase 218 and aqueous phase 216 may be separated from heated combined solution 214. At least a portion (preferably greater than 50%) of non-aqueous phase 118 or 218 may be provided to a distillation unit to recover at least a portion of the furfural as overhead product 120 while leaving a majority (greater than 50%) of non-aqueous phase 118 or 218 comprising boronic acid and water-insoluble solvent as bottom product 122. Suitably, additionally, or alternatively, permeation or affinity separation (not shown) can be used to recover at least a portion of the produced furfural.

Referring to FIGS. 1 and 2, bottom product 122 comprises at least a portion of water-insoluble boronic acid and water-insoluble solvent from extraction solution 104. Optionally, at least a portion of bottom product 122 can be recycled (i.e., reused) as part of extraction solution 104. That is, optionally, extraction solution 104 can comprise at least a portion of bottom product 122, including at least 20 wt.%, preferably at least 50 wt.%, more preferably at least 70 wt.%, and most preferably at least 90 wt.% or at least 95 wt.%.

In addition to water-insoluble solvent and water-insoluble boronic acid, bottom product 122 or 222 can further comprise an amount of water-soluble solvent (such as, at least 0.1 wt.% to 20 wt.%, preferably 0.5 wt.% to 10 wt.%, and more preferably 1 wt.% to 5 wt.%). If an amount of bottom product 122 or 222 is recycled for use as part of extraction solvent 104, at least some of the water-soluble solvent in bottom product 122 or 222 can be in aqueous phase 108, along with other components from bottom product 122 or 222. That is, aqueous phase 108 can comprise (i) water-soluble solvent, (ii) water-insoluble solvent, and (iii) water-insoluble boronic acid. Optionally, aqueous phase 108 may be further processed (not shown) to recover at least one of such water-soluble solvent, water-insoluble solvent, and water-insoluble boronic acid. Suitable further processing of aqueous phase 108 may include adsorption, such as if the water-soluble solvent comprises sulfolane, then activated carbon may be used to adsorb a portion of the sulfolane. Such adsorption bed may also be used recover a portion of water-insoluble solvent and water-insoluble boronic acid from aqueous phase 108.

Referring to FIGS. 1 and 2, aqueous phase 116 or 216 also comprises furfural from second combined solution 124 or 214, respectively, and may be further processed to recover at least a portion of that furfural using suitable methods known to one of ordinary skills. For instance, aqueous phase 116 or 216 may be provided to a distillation unit to recover the furfural as overhead product 126 (in vapour form) which comprises water and furfural since furfural forms a heterogeneous azeotrope with water. By water-furfural heterogeneous azeotrope, we mean a mixture of water and furfural that are co-distilled at about 2:1 weight ratio as top product and separate into a water-rich and a furfural-rich phase upon condensation. The furfural in overhead product 126 may be optionally recovered by cooling overhead product 126 to allow it to condense from vapour phase to liquid phase and separate into an aqueous phase (not shown) and a non-aqueous phase comprising more furfural than the aqueous phase of condensed overhead product 126. Accordingly, process 100 or 200 can optionally comprise cooling at least a portion of overhead product 126 to temperature Tc to allow such condensation and separation. Preferably, Tc is less than 100 °C at 1 atmospheric pressure, preferably less than 80 °C, and more preferably less than 60 °C.

Aqueous phase 130 comprises (i) water in a range from 50 to 95 wt.%, preferably about 92wt% and (ii) at least 1 wt.%, preferably at least 5 wt.% and most preferably about 8 wt.% furfural, and non-aqueous phase 132 comprises (i) furfural in a range from 50 to 95 wt.% and (ii) at least 5 wt.% water, preferably 6 wt.%. Aqueous phase 130 and non-aqueous phase 132 may be separated for further furfural recovery using suitable separation methods such as liquid-liquid separation as described herein. Referring to FIGS. 1 and 2, bottom product 128 from such distillation comprises water and water-soluble solvent originally in conversion solution 112 and is acidic (i.e., has pH less than 7). Preferably, as shown, at least a portion (at least 50%, preferably at least 75%) of bottom product 128 may optionally be recycled (i.e., reused) as part of conversion solution 112.

Alternatively or additionally to providing aqueous phase 116 to a distillation unit for further processing to recover at least a portion of the residual furfural in aqueous phase 116, the residual furfural may be extracted with a water-insoluble solvent as known to one of ordinary skill, including those described herein. Optionally, such water-insoluble solvent may comprise at least a portion of bottom product 122 or 222. That is, at least a portion of bottom product 122 or 222 can be used in extraction of furfural in aqueous phase 116.

As described, referring to FIGS. 1 and 2, aspects of processes 100 and 200 allow for recycling of at least a portion (preferably greater than 50%) of (i) bottom product 122 in extraction solution 104 and/or (ii) bottom product 128 in conversion solution 112. Such recycling reduces the consumption of costly chemicals, particularly water-insoluble solvent and water-insoluble boronic acid of extraction solution 104 and/or water-soluble solvent and inorganic acid of conversion solution 112.

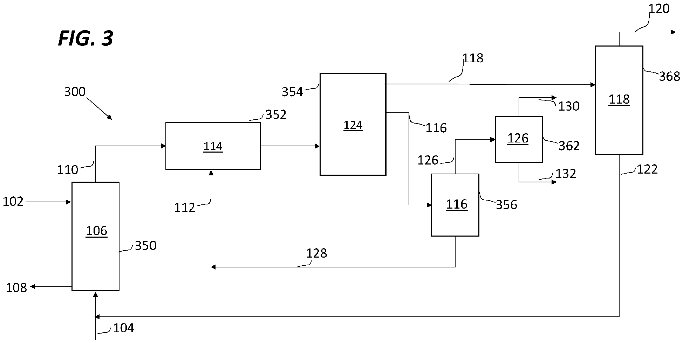

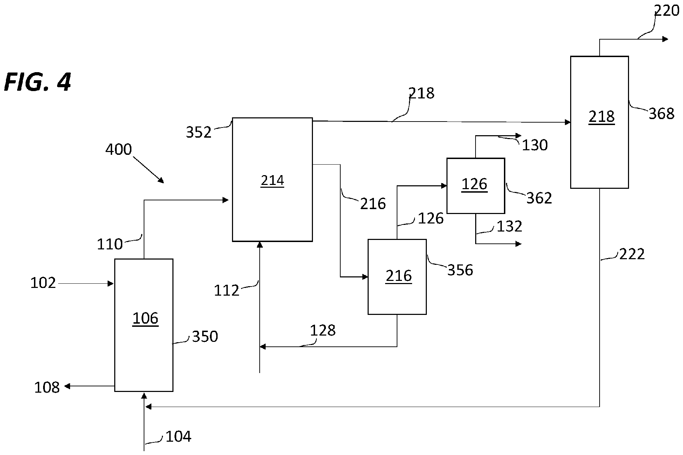

FIG. 3 illustrates one exemplary suitable embodiment of system 300 for producing furfural from xylose in accordance with this disclosure, such as process 100. FIG. 4 similarly illustrates one exemplary suitable embodiment of system 400 for performing certain aspects of the processes described herein, such as process 200. As shown in FIGS. 3 and 4, xylose-containing solution 102 and extraction solution 104 are provided to and combined in extraction unit 350 to form first combined solution 106. Extraction unit 350 is designed to perform liquid-liquid extraction, preferably in counter-current, and perform separation to separate non-aqueous phase 110 as described above. As shown in FIGS. 3 and 4, system 300 allows for the steps of forming first combined solution 106 and separating non-aqueous phase 110 to be carried out at least partially (and/or fully) together via extraction unit 350. Extraction unit can be performed in co-current flow and preferably counter-current flow.

Extraction unit 350 can be operated isothermally for a desired amount of time at a constant temperature. Additionally or alternatively, extraction unit 350 can be operated with a temperature gradient for a desired amount of time by providing xylose-containing solution 102 at a temperature that is at least 5 °C, at least 10 °C, at least 15 °C, or at least 20 °C, higher than the temperature of extraction solution 104. If extraction unit 350 is operated in counter-current mode with a temperature gradient, such operation can combine a higher extraction rate associated with the warmer section that is in close proximity with the inlet of extraction unit 350 for xylose-containing solution 102 (which translates to higher concentration of xylose in the portion of first combined solution 106 at that location) with low loss of extraction solution 104 in the water-rich effluent in the cooler section in close proximity with an outlet for aqueous phase 108.

Referring to FIGS. 3 and 4, aqueous phase 108 and non-aqueous phase 110 are separated via extraction unit 350. Non-aqueous phase 110 comprises at least 20 mol% (and preferably more than 50 mol%) of the xylose in the xylose containing solution 102 initially provided to form first combined solution 106. The processes described herein, such as process 100 in FIG. 1 which can be performed with system 300 in FIG. 3, can be designed to have at least 90% selectivity for xylose, which means in such an embodiment, non-aqueous phase 110 comprises at least 90% and aqueous phase 108 comprises less than 10% of the xylose originally in xylose-containing solution 102.

Referring to FIGS. 3 and 4, non-aqueous phase 110 is provided to dehydration unit 352. Conversion solution 112 is also provided to dehydration unit 352, and non-aqueous phase 110 and conversion solution 112 are combined in unit 352 to form second combined solution 114 or 214, respectively. Referring to FIGS. 3 and 4, second combined solution 114 and 214, respectively, are heated while preferably being mixed at least a portion (or substantially all) of the time being heated in dehydration unit 352 by a suitable method and corresponding component(s) as described above. In one embodiment, second combined solution 114 and 214 are heated to a temperature of at least 100 °C in dehydration unit 352 to convert xylose-diboronate ester to furfural.

Referring to FIG. 3, second combined solution 114 is heated to temperature Th in dehydration unit 352 where second combined solution 114 consists essentially of a homogeneous liquid phase, the duration of which and optional mixing are in accordance with the descriptions provided herein. Such heated second combined solution 114 is provided to cooling unit 354 for cooling to temperature Tc, thereby forming cooled second combined solution 124 which comprises an aqueous phase comprising water, water-soluble solvent, and furfural, and a non-aqueous phase comprising water-insoluble boronic acid, water-insoluble solvent, and furfural. At least a portion of such aqueous and non-aqueous phases of cooled second combined solution 124 can be separated as stream 116 and 118, respectively, using suitable methods such as those described herein, including liquid-liquid separation.

Referring to FIG. 4, second combined solution 214 is heated in dehydration unit 352 to a temperature below temperature Th, where heated second combined solution 214 comprises an aqueous phase comprising water, water-soluble solvent, and furfural, and a non-aqueous phase comprising water-insoluble boronic acid, water-insoluble solvent, and furfural. At least a portion of such aqueous and non-aqueous phases of second combined solution 214 can be separated as stream 216 and 218, respectively, using suitable methods such as those described herein, including liquid-liquid separation. In system 400, dehydration unit 352 is also capable of performing liquid-liquid separation (or extraction) to separate aqueous phase 216 and non-aqueous phase 218 from second combined solution 214.

### Further processing and/or furfural recovery options

Referring to FIGS. 3 and 4, aqueous phases 116 and 216, respectively, are provided to distillation unit 356, which is preferably operated under conditions configured to recover residual furfural contained in aqueous phases 116 and 216 that was produced in dehydration unit 352. For instance, unit 356 can be operated slightly above ambient pressure (e.g., 1 atm) and with a top temperature of unit 356 at about 98 °C to 100 °C. At least a portion of the furfural in aqueous phases 116 and 216 is recovered as top product 126, which comprises water and furfural. Bottom product 128 is acidic (i.e., has pH less than 7) and comprises an amount of water-soluble solvent from conversion solution 112. Preferably, as shown, at least a portion (at least 50%, preferably at least 75%) of bottom product 128 is recycled (i.e., reused) as part of conversion solution 112.

Referring to FIGS. 3 and 4, overhead product 126 of distillation unit 356 comprises water and furfural since furfural forms a heterogeneous azeotrope with water. As shown, overhead product 126 is provided to cooling unit 362 to be cooled to temperature Tc, which is the temperature at or below which the water/furfural azeotrope in overhead product 126 (exiting unit 356 at a temperature in a range from 98 °C -100 °C at 1 atmosphere) to condense and separate into aqueous phase 130 comprising (i) water in a range from 50 to 95 wt.%, preferably about 92% and (ii) at least 5 wt.%, preferably about 8 wt.% furfural, and non-aqueous phase 132 comprising (i) furfural in a range from 50 to 95 wt.% and (ii) at least 5 wt.% water, preferably 6 wt.%. Cooling unit 362 is preferably capable of performing liquid-liquid separation to separate aqueous phase 130 and non-aqueous phase 132 from each other.

Referring to FIGS. 3 and 4, non-aqueous phase 118 or 218, respectively, is provided to distillation unit 368 for recovery of furfural in overhead product stream 120. As shown, at least a portion (at least 50% or substantially all) of bottom product stream 122 can be recycled (or reused) as part of extraction solvent 104.

Optionally, in addition to or as an alternative to using distillation for unit 356 to remove furfural from aqueous phase 116 to provide bottom product stream 128, unit 356 can comprise a suitable liquid-liquid extraction process using a suitable water-insoluble solvent to extract furfural into the water-insoluble solvent. Such water-insoluble solvent for use in this optional aspect of unit 356 can comprise at least a portion of bottom product stream 122 or 222.

Preferably, embodiments of the processes and systems as described herein are carried out or operated continuously for an amount of time, such as at least 6 hours.

### Xylose back extraction

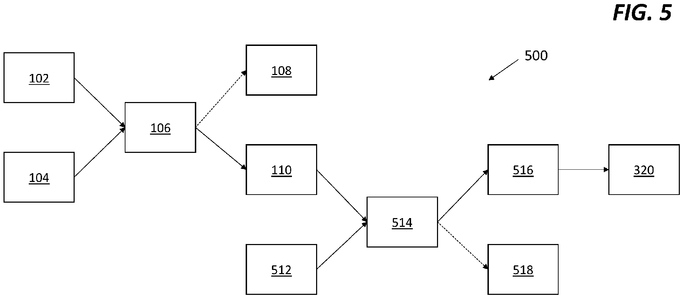

FIG. 5 shows process 500 which can be used to isolate xylose from xylose-containing solution 102 for other further processing, including subsequent evaporation of water to produce the xylose in powder form. Referring to FIG. 5, nonaqueous phase 110 is combined with back extraction solution 512 to form BA₂X-containing solution 514 and to convert the xylose-diboronate esters (BA₂X) back into xylose. Back extraction solution 512 is preferably an aqueous and acidic solution with a pH of less than or equal to 6, preferably less than or equal to 6, and more preferably less than or equal to 2. BA2X-containing solution 514 preferably comprises back extraction solution 512 and non-aqueous phase 110 in a ratio by weight of in a range from 0.1 to 10, preferably from 0.3 to 5, and more preferably from 1 to 3, respectively. BA₂X-containing solution 514 may be heated to a temperature in a range from 20 to 150 °C, preferably from 40 to 120 °C, and more preferably from 60 to 100 °C, for an amount of time of at least 1 minute, preferably at least 10 minutes, and more preferably at least 20 minutes. Optionally, BA₂X-containing solution 514 may be mixed at least a portion of the time it is heated.

In BA₂X-containing solution 514, the xylose-diboronate esters react with water, which converts the xylose-diboronate esters to into boronic acid and xylose, which is more soluble in aqueous phase than non-aqueous phase. As more xylose-diboronate esters dissociate into xylose, an aqueous phase comprising xylose and water and a non-aqueous phase comprising boronic acid and water-insoluble solvent from extraction solution 104 begin to form, wherein the concentrations of water and xylose in such aqueous phase is higher than those in such non-aqueous phase of solution 514. Such aqueous and non-aqueous phases can be recovered from solution 514 as aqueous phase 516 and non-aqueous phase 518 using suitable separation methods, such as those described herein.

Aqueous phase 516 is substantially free of contaminants potentially present in xylose-containing solution 102 which may have presented challenges to isolation of the xylose from solution 102. For instance, if xylose-containing solution 102 were a hydrolysate, evaporation of water from solution 102 would have left a solid product containing xylose along with other contaminants, particularly with the amount of xylose being a small percentage of the solid product. In aqueous-phase 516, however, the amount of xylose would be significantly more than other contaminants from xylose-containing solution 102 because of the selectivity of boronic acid to xylose to form xylose-diboronate esters, which are extracted with extraction solution 104. At least a portion of the xylose in aqueous phase 516 may be recovered in solid or powder form using methods known to one of ordinary skill, including evaporation of water.

If process 500 is selected to recover xylose in addition or as an alternative to furfural production as described with FIGS. 1-4, extraction solution 104 may be designed to comprises a water-insoluble solvent that has moderate affinity for the xylose-diboronate esters (i.e., the BA₂X has moderate solubility in the selected water-insoluble solvent) (e.g., heptane instead of toluene) to balance extraction and back extraction. That is, the higher the affinity for xylose-diboronate esters (meaning more of the xylose-diboronate ester resides in the water-insoluble solvent as compared to another solvent that could be selected), the greater amount of back conversion solution 512 is needed to drive the reaction to the xylose.

### EXAMPLES

Embodiments of the processes and systems described herein can be further illustrated by the following exemplary, non-limiting examples

### Example 1 - xylose extraction

In Example 1, nine experiments were conducted with different water-insoluble solvent and pH as shown in Table 4 below. General experimental conditions for these nine experiments include:
- xylose-containing solution: 150 mL solution containing D-xylose (350 mM) in water that was acidified to various pH with H₂SO₄ and
- extraction solution: 150 mL solution containing phenyl boronic acid (PBA) in a water-insoluble solvent; the concentration of PBA and type of water-insoluble solvent are provided in Table 4 below
- the xylose-containing solutions and extraction solutions of Example 1 were each mixed in a 500 mL Erlenmeyer flask, provided with a cap, and stirred at room temperature for 2 hours, which results in an aqueous phase and a non-aqueous phase in the flasks. Subsequently, the two phases of each experiment were separated and the resulting concentrations of xylose (X) and xylose diboronate ester (BA₂X) were quantified in each phase using ¹H-NMR analysis in the presence of internal standard as described below. The quality of extraction is then reported as molar yield of BA₂X found in the organic phase in Table 4 below.

The aqueous phases and non-aqueous phases from Example 1 were analysed by means of 1H-NMR using a 400 MHz Bruker spectrometer. The aqueous phases were measured in a 1:1 H₂O/D₂O mixture (in which D2O is deuterated water also called heavy water or heavy water used for the analysis) with Trimethylsilyl propanoic acid (TMSP) as the internal standard and the non-aqueous phases were measured in a 1:1 mixture of toluene and toluene-d8 with dioxane as the internal standard. These mixtures of aqueous and non-aqueous phases being analysed are composed by equal volumes (250 µL) of the particular aqueous or non-aqueous phase and the deuterated solvent, containing the standard.

**Table 4**

| Extraction efficiency at various conditions (Xylose concentration in water 350 mM, room temperature.) | | | | |
|---|---|---|---|---|
| | **Water-insoluble solvent** | **PBA (mM)** | **pH** | **Extracted Xylose (%mol)** |
| 1 | toluene | 700 | 1 | 84 |
| 2 | toluene | 350 | 1 | 47 |
| 3 | toluene | 1050 | 1 | 95 |
| 4 | toluene | 700 | 3 | 83 |
| 5 | toluene | 700 | 6 | 84 |
| 6 | toluene | 700 | 9 | 83 |
| 7 | 2-methyl naphthalene | 700 | 1 | 89 |
| 8 | n-heptane | 700 | 1 | 55 |
| 9 | 1-octanol | 700 | 1 | 30 |

The experiments of Example 1 show recovery of xylose using toluene, 2-methylnaphthalene, n-heptane, and octanol as water-insoluble solution, with PBA as the water-insoluble boronic acid.

In experiments 1 and 4-9, the molar ratio of boronic acid to xylose was 2:1. Experiments 3 and 4 demonstrate a potential correlation between extraction efficiency and ratio of boronic acid to xylose, particularly that a higher molar ratio of boronic acid can produce a higher extraction rate (experiment 3) while a lower molar ratio of boronic acid also results in extraction of xylose but at a lower rate (experiment 4).

In addition, these experiments show that embodiments of the processes and systems described herein allow for xylose extraction from acidic and basic xylose-containing solutions. The experiments also demonstrate that embodiments of the processes and systems described herein allow for xylose extraction using aromatic solvents and aliphatic solvents, although the aromatic solvents (toluene and 2-methylnaphthalene) provide a better rate of extraction than the aliphatic solvents (n-heptane and octanol). The non-aqueous phase of experiments 1-9 of Example 1 can either be back extracted to xylose as described above (particularly with respect to FIG. 5), and/or further combined with a conversion solution to produce furfural (dehydration reaction) as described herein (such as conversion solution 112). Example 2 below shows experiments of such dehydration reaction.

### Example 2 - Xylose dehydration

In Example 2, experiments were conducted under different conditions as set forth in Table 5 below. General experimental conditions for the 12 experiments include:
- second combined solution: 2.5 mL of a representative non-aqueous phase containing 290 mM of BA₂X in various water-insoluble solvents was added to an amount of a representative conversion solution with (i) 2.5 mL of acidic water having a pH of 1 (using H₂SO₄) and (ii) various amounts water-soluble solvent in a ratio of water-insoluble solvent to acidic water to water-soluble solvent of 1:1:X.

The various second combined solutions as shown in Table 5 below were provided to a heavy glass wall cylindrical pressure vessel having a total volume 30 mL, which was sealed with a Teflon cap, and heated at 180 °C for various amounts of time shown in Table 5.

The pressure vessel for each experiment was cooled, and the liquid product was separated into a bottom aqueous phase and a top toluene-rich phase. The resulting concentrations of residual xylose diboronate ester (BA₂X), xylose (X) and furfural (F) were quantified in each phase using ¹H-NMR analysis in the presence of internal standard as described above. The sum of the molar yields of furfural found in the aqueous and organic phase for each experiment are shown below in Table 5. In addition, Table 5 shows the conversion mol%, which indicates the mol% of free xylose or BA₂X that was converted to furfural.

**Table 5**

| BA₂X dehydration at various conditions (initial concentration in the aromatic solvent 290 mM, 180 °C, 1:1:X volume ratio of water-insoluble solvent: acidic water : water-soluble solvent). | | | | | | |
|---|---|---|---|---|---|---|
| | Water-insoluble solvent | Water-soluble solvent | X (volume ratio of water-soluble solvent) | Time (h) | Conversio n (mol%) | Furfural yield (mol%) |
| 1 | toluene | DMSO | 0.1 | 2 | 55 | 37 |
| 2 | toluene | DMSO | 0.5 | 2 | 74 | 58 |
| 3 | toluene | DMSO | 1 | 2 | 86 | 78 |
| 4 | toluene | DMSO | 1 | 3 | 100 | 90 |
| 5 | nitrobenzene | DMSO | 1 | 2 | 100 | 95 |
| 6 | 2-MN | DMSO | 1 | 3 | 100 | 87 |
| 7 | toluene | sulfolane | 1 | 0.5 | 31 | 29 |
| 8 | toluene | sulfolane | 1 | 2 | 80 | 76 |
| 9 | toluene | sulfolane | 1 | 3.5 | 100 | 95 |
| 10 | 1-MN | sulfolane | 1 | 3 | 100 | 89 |
| 11 | toluene | GVL | 1 | 4 | 100 | 65 |
| 12 | toluene | dioxane | 1 | 3 | 100 | 60 |

The experiments of Example 2 show furfural production in accordance with embodiments of the processes and systems described herein. Such furfural yields can be improved with at least one of (i) using a 1:1:1 volume ratio of water-insoluble solvent: acidic water : water-soluble solvent (see experiments 1 and 2 as compared to experiment 3 in which lower yields were observed with lower ratio of water-soluble solvent), (ii) selecting a longer reaction time (see experiment 3 vs. 4 in which lower furfural yields were observed with shorter reaction time, or experiments 7 - 9, in which similar correlation between yields and reaction time was observed), and (iii) solvent selection (see experiment 3 vs. 5 in which toluene vs. benzene was used with DMSO, or experiments 10 - 12 in which the combination of 1-MN and sulfolane is more effective than toluene and GVL dioxane, all of which indicates aromatic water-insoluble solvent can be preferred for use with DMSO or sulfolane as water-soluble solvent (vs. GVL or dioxane).

In Example 2, additional experiments 13 - 19 as shown in Table 6 were carried out to demonstrate the desirable effect of converting the BA₂X to furfural under conditions that allow the formation all the reaction components to merge into a single phase during the reaction (e.g., heating the second combined solution to temperature Tₕ). These experiments were carried out under the same general conditions as described above for experiments 1 - 12 of Example 2 with the representative second combined solution comprising toluene-water-sulfolane mixture in 1:1:1 volume ratio and PBA but with varying either the reaction temperature or the PBA concentration to switch between monophasic and bi-phasic conditions, which was witnessed by visual inspection. The variation in PBA concentration was executed by replacing the xylose-diboronate ester (PBA₂X) with an equal molar amount of free xylose and a varying molar amount of free PBA.

**Table 6**

| Xylose dehydration at various conditions (initial concentration in water 290 mM, 1:1:1 volume ratio toluene-sulfolane-water pH = 1 solvent system, full conversion reached in all cases). | | | | | |
|---|---|---|---|---|---|
| | **T (°C)** | **PBA (mM)** | **Time (h)** | **# phases at reaction conditions** | **Furfural yield (mol%)** |
| 13 | 180 | 6 | 3.5 | 2 | 73 |
| 14 | 180 | 70 | 3.5 | 1 | 92 |
| 15 | 180 | 300 | 3.5 | 1 | 96 |
| 16 | 180 | 600 | 3.5 | 1 | 95 |
| 17 | 165 | 2000 | 8 | 1 | 89 |
| 18 | 160 | 600 | 9 | 2 | 71 |
| 19 | 150 | 2000 | 14 | 2 | 65 |

Similar to above, experiments 13 - 19 show furfural production in accordance with embodiments of the processes and systems described herein. Such furfural yield can be improved by heating second combined solution to temperature Tₕ at or above which the second combined solution consists essentially of a homogenous phase. For instance, experiments 14 - 17 show that furfural production yields of at least about 90% were observed under conditions that allow the two liquid phases observed at room temperature fuse into a single liquid phase at reaction temperature as compared to when the representative second combined solution has two phases at reaction temperature.

### Example 3 - recycling of certain bottom products

We first analysed one non-aqueous phase comprising methylnaphthalene (MN) (such as one potential embodiment of non-aqueous phase 118 or 218 of FIGS. 1-4, respectively) and one aqueous phase comprising water and sulfolane (S) (such as one potential embodiment of aqueous phase 116 or 216 of FIGS. 1 - 4, respectively) that are separated in the decanter after the dehydration reaction (such as those in Example 2 and/or one embodiment of second combined solution 114 or 214 of FIGS. 1-4, respectively). Table 7 below reports the distribution of the main components over the two phases at room temperature. The distribution between polar and apolar phases is reported as ratio of concentrations K_{d} and ratio of absolute amounts Pₘₒₗ. It should be noticed that volume of the polar phase of water + sulfolane is twice as large as that of the apolar phase, which results in Pₘₒₗ being twice as large as K_{d}.

**Table 7**

| Partition of components in Example 3 measured at room temperature | | | | | | |
|---|---|---|---|---|---|---|
| | [Conc]_{water+S} (mM) | [conc]_{MN} (mM) | Wt.%_{water+S} | Wt.%_{MN} | K_{d} [X] | Pₘₒₗ |
| Furfural | 103 | 144 | 0.88 wt.% | 1.3 wt.% | 0.72 | 1.43 |
| water | 27,600 | 92 | 44 wt.% | 0.15 wt.% | 300 | 601 |
| Sulfolane | 5,100 | 370 | 54 wt.% | 4.0 wt.% | 27.3 | 13.8 |
| PBA | 125 | 340 | 1.3 wt.% | 3.7 wt.% | 0.37 | 0.74 |
| 1-MN | 3 | 7,000 | 0.04 wt.% | 91 wt.% | 0.0004 | 0.0009 |

Table 7 shows that the addition of sulfolane improves the solubility of both MN (water-insoluble solvent) and PBA in the aqueous phase. Accordingly, the aqueous phase that consists essentially of water and sulfolane also contains traces of furfural (<1 wt.%), PBA (<1.3 wt.%) and MN (<0.1wt%). Optionally, furfural can be recovered as described, for instance as overhead product 126 by providing the polar phase to unit 356 in FIGS. 3 or 4, and the MN and PBA remaining, such as in bottom product 128 of FIGS. 1-4, can be recycled to the dehydration reaction as part of the conversion solution.

The non-aqueous phase that is rich in MN (water-insoluble solvent), meaning greater concentration of MN as compared to the aqueous phase, contained much (41%) of the overall furfural produced and initial PBA (boronic acid). As described, furfural can be recovered from this non-aqueous phase in an overhead product of a distillation process. The remaining bottom product (e.g., 122 or 222) comprising MN and PBA can be recycled as part of the extraction solution. Because the non-aqueous phase also contained some sulfolane (water-soluble solvent, 4wt%), which may get lost in the aqueous phase of first combined solution if the bottom product comprising MN and PBA is recycled as described. Optional further processing of the aqueous phase of first combined solution (such as aqueous phase 108 of FIGS. 1-4) may be employed to reduce such potential loss.

As described, the present disclosure provides processes and systems to produce furfural from an aqueous xylose-containing solution without isolation of xylose in dry form, which can be costly. In particular, certain embodiments of the processes and systems described herein (such as process 100 and system 300) are capable of and can be designed to produce such furfural at a relatively higher yield of at least 80 mol%, preferably at least 85 mol%, more preferably at least 90 mol%, and most preferably at least 95 mol% (meaning at least 80, 85, 90, or 95 mol% of xylose is converted to furfural) without isolation of xylose in dry form. Although other embodiments (such as process 200 and system 400) may not provide such high yields, they nonetheless can still provide various benefits described herein. One of ordinary skills can design various embodiments as described herein to achieve the desired yields in light of various factors (such as energy demands, equipment costs, etc.).

Embodiments of the processes and systems described herein allow for less formation of undesirable by-products (such as humins) from the various reactants and/or furfural degradation, which allows for furfural production with marginal fouling of equipment and marginal contamination of various product and solvent streams. The selectivity of water-insoluble boronic acid for xylose (in contrast with other sugars such as glucose and mannose) in first combined solution 106 allows for extraction of xylose as described while the majority (greater than 50%) of other sugars remain in solution 108 after non-aqueous phase 110 comprising xylose-diboronate esters is separated. Non-aqueous phase 110 comprising less contaminants (i.e., non-xylose components such as other sugars and lignin) means less contaminants that are subject to dehydration reactions, such as those described for second combined solution 114, 124, and/or 214, and/or carried out in unit 352, which would degrade these contaminants to humins and other fouling components.

Furthermore, various embodiments of the processes and systems describe herein enable recovery of furfural using distillation of water-insoluble components in non-aqueous phase 118 or 218, which is, broadly speaking, simpler as compared to distillation of an aqueous solution containing furfural. For instance, various embodiments described herein allow one of ordinary skill to optionally select a water-insoluble solvent with a boiling point above the boiling point of furfural. Such solvent selection can further reduce energy demands of the corresponding process or system by enabling recovery via distillation of the minor component, furfural, as an overhead product (such as 120 or 220) of the distillation column instead of distilling the major component, the water-insoluble solvent.

Moreover, optional recovery of furfural from aqueous phase 116 or 216 is made possible. It can include post-distillation processing to separate the furfural and water from the water-furfural azeotrope that forms in overhead product 126. However, it can also consist of extraction using at least a portion of bottom product 122 or 222 comprising water-insoluble solvent and boronic acid.

As noted above, the processes and systems described herein can be carried out in solutions without needing the xylose to be in solid form for furfural production, which can allow for a simplified approach of extracting xylose as xylose-diboronate ester into a non-aqueous phase, dehydration of that non-aqueous phase to produce furfural, and recovery of furfural. Nevertheless, embodiments of the processes and systems described herein can be designed to isolate xylose in solid form.

## Claims

1. A method for producing furfural comprising:
a. providing a xylose-containing solution comprising xylose in an amount of greater than or equal to 0.5 wt.%, wherein the xylose-containing solution is an aqueous solution;
b. providing an extraction solution comprising a water-insoluble boronic acid (BA: R-B(OH)₂) and a water-insoluble solvent;
c. combining the xylose-containing solution with the extraction solution to provide a first combined solution, wherein the ratio of boronic acid to xylose in the first combined solution is greater than 1:1 molar, respectively, and wherein the first combined solution comprises a first aqueous phase and a first non-aqueous phase, said non-aqueous phase comprising at least a portion of the xylose as xylose-diboronate ester (BA₂X);
d. separating at least a portion of the first non-aqueous phase from the first combined solution;
e. providing a conversion solution comprising a water-soluble solvent and water, said conversion solution has a pH of less than or equal to 4;
f. combining at least a portion of the non-aqueous phase from (d) with the conversion solution in a ratio of conversion solution to the non-aqueous phase in a range from 0.1 to 10.0 by weight, respectively, to form a second combined solution;
g. heating the second combined solution to a temperature Tₕ at or above which the second combined solution consists essentially of a homogeneous liquid phase, wherein such heating converts at least a portion of the xylose-diboronate ester into furfural,
h. cooling down the heated second combined solution such that the cooled second combined solution comprises (i) a second aqueous phase comprising water, water-soluble solvent, and furfural and (ii) a second non-aqueous phase comprising water-insoluble solvent, water-insoluble boronic acid, and furfural; and separating at least a portion of the second non-aqueous phase from the cooled second combined solution; and
i. recovering at least a portion of the furfural in the second non-aqueous phase.

2. A method for producing furfural comprising:
a. providing a xylose-containing solution comprising xylose in an amount of greater than or equal to 0.5 wt.%, wherein the xylose-containing solution is an aqueous solution;
b. providing an extraction solution comprising a water-insoluble boronic acid (BA: R-B(OH)₂) and a water-insoluble solvent;
c. combining the xylose-containing solution with the extraction solution to provide a first combined solution, wherein the ratio of boronic acid to xylose in the first combined solution is greater than 1:1 molar, respectively, and wherein the first combined solution comprises a first aqueous phase and a first non-aqueous phase, said non-aqueous phase comprising at least a portion of the xylose as xylose-diboronate ester (BA₂X);
d. separating at least a portion of the first non-aqueous phase from the first combined solution;
e. providing a conversion solution comprising a water-soluble solvent and water, said conversion solution has a pH of less than or equal to 4;
f. combining at least a portion of the non-aqueous phase from (d) with the conversion solution in a ratio of conversion solution to the non-aqueous phase in a range from 0.1 to 10.0 by weight, respectively, to form a second combined solution;
g. heating the second combined solution to a temperature of greater than or equal to 100°C to convert at least a portion of the xylose-diboronate ester into furfural, wherein the heated second combined solution comprises a second aqueous phase comprising water, at least a portion of the water-soluble solvent from the conversion solution, and furfural and (ii) a second non-aqueous phase comprising at least a portion of water-insoluble solvent and water-insoluble boronic acid from the extraction solution, and furfural,
h. separating at least a portion of the second non-aqueous phase from the cooled second combined solution; and
i. recovering at least a portion of the furfural from the non-aqueous phase.

3. The method of any one of claims 1 to 2 wherein step (i) comprises providing at least a portion of the second non-aqueous phase from (h) to a distillation process to recover an overhead product comprising furfural and a bottom product comprising water-insoluble solvent and water-insoluble boronic acid.

4. The method of claim 3 further comprising providing at least a portion of the bottom product for use as part of the extraction solution.

5. The method of claim 4 wherein at least a portion of the first aqueous phase in (c) comprises water-soluble solvent, water-insoluble solvent, and water-insoluble boronic acid, said method further comprising:
separating at least a portion of the first aqueous phase in (c) from the first combined solution; and
further processing at least a portion of the separated first aqueous phase to recover at least one of water-soluble solvent, water-insoluble solvent, and water-insoluble boronic acid.

6. The method of any one of claims 1 - 5 further comprising performing at least a portion of steps (c) and (d) in a liquid-liquid extraction unit in counter-current operation, wherein the xylose-containing solution is provided at a higher temperature than the temperature of the extraction solution.

7. The method of any one of claims 1 - 6 further comprising providing at least a portion of the second aqueous phase from (h) to a distillation process to recover an overhead product comprising water and furfural and an acidic bottom product comprising water and water-soluble solvent, wherein the bottom product has a pH of less than 7.

8. The method of claim 7 further comprising providing at least a portion of the acidic bottom product for use as part of the conversion solution.

9. The method of any one of claims 3-6 further comprising providing at least a portion of the second aqueous phase from (h) to a solvent-extraction process to recover furfural, wherein at least a portion of solvent used in the extraction process comprises the distillation bottom product comprising water-insoluble solvent and water-insoluble boronic acid.

10. The method of any one of claims 1 to 9 wherein the xylose-containing solution is a hydrolysate.

11. The method of any one of claims 1 - 10 wherein the water-insoluble boronic acid has up to 5 wt.% solubility in water at 20 °C.

12. The method of any one of claims 1 - 11 wherein the water-insoluble boronic acid is selected from the group consisting of phenylboronic acid, 4-biphenylboronic acid, 4-butylphenyl boronic acid, 4-tert-Butylphenyl boronic acid, 4-ethylphenyl boronic acid, 2-naphthylboronic acid, naphthalene-1-boronic acid, o-tolylboronic acid, m-tolylboronic acid, (2-methylpropyl) boronic acid, butylboronic acid, octylboronic acid, phenethyl boronic acid, cyclohexyl boronic acid, and any combination thereof.

13. The method of any one of claims 1 - 12 wherein the water-insoluble solvent has up to 5 wt.% solubility in water at 20°C.

14. The method of any one of claims 1 - 13 wherein the water-insoluble solvent is selected from the group consisting of benzoic acid, cresol (m), di-isopropyl ether, terephthalic acid, diethylene glycol diethyl ether, anisole, salicylic acid, 2,6 xylenol, 4Et-phenol, toluene, benzofuran, ethylbenzene, octanoic acid, 1-methylnaphtalene, nitrobenzene, guaiacol, heptane, 1-octanol, and methylisobutyl ketones, an any combination thereof.

15. The method of any one of claims 1 - 14 wherein at least one of the water-insoluble boronic acid and water-insoluble solvent has a boiling point higher than that of furfural, preferably at least 5 °C higher.

16. The method of any one of claims 1 - 15 wherein the water-soluble solvent has a logP (octanol-water partition co-efficient) in a range from (-3) to 0.

17. The method of any one of claims 1 - 16 wherein the water-soluble solvent is selected from the group consisting of dimethyl sulfoxide (DMSO), diglyme, sulfolane, gamma butyrolactone, succinic acid, nMe-acetamide, dioxane, nMe-pyrrolidone, gamma valerolactone, acetone, acetic acid, and any combination thereof.

18. The method of any one of claims 1-17 wherein the water-soluble solvent has a boiling point higher than that of water, preferably at least 5 °C higher.

## Patentansprüche

1. Verfahren zum Produzieren von Furfural, umfassend:
a. Bereitstellen einer xylosehaltigen Lösung, umfassend Xylose in einer Menge von größer als oder gleich 0,5 Gew.-%, wobei die xylosehaltige Lösung eine wässrige Lösung ist;
b. Bereitstellen einer Extraktionslösung, umfassend eine wasserunlösliche Boronsäure (BA: R- B(OH)₂) und ein wasserunlösliches Lösungsmittel;
c. Kombinieren der xylosehaltigen Lösung mit der Extraktionslösung, um eine erste kombinierte Lösung bereitzustellen, wobei das Verhältnis von Boronsäure zu Xylose in der ersten kombinierten Lösung jeweils größer als 1:1 molar ist, und wobei die erste kombinierte Lösung eine erste wässrige Phase und eine erste nicht wässrige Phase umfasst, die nicht wässrige Phase umfassend mindestens einen Anteil der Xylose als Xylose-Diboronatester (BA₂X);
d. Abscheiden von mindestens einem Anteil der ersten nicht wässrigen Phase von der ersten kombinierten Lösung;
e. Bereitstellen einer Umwandlungslösung, umfassend ein wasserlösliches Lösungsmittel und Wasser, wobei die Umwandlungslösung einen pH-Wert von kleiner als oder gleich 4 aufweist;
f. Kombinieren von mindestens einem Anteil der nicht wässrigen Phase aus (d) mit der Umwandlungslösung in einem Verhältnis von Umwandlungslösung zu der nicht wässrigen Phase in einem Bereich von jeweils 0,1 bis 10,0 nach Gewicht, um eine zweite kombinierte Lösung auszubilden;
g. Erhitzen der zweiten kombinierten Lösung auf eine Temperatur Tₕ bei oder über der die zweite kombinierte Lösung im Wesentlichen aus einer homogenen flüssigen Phase besteht, wobei ein solches Erhitzen mindestens ein Anteil des Xylose-Diboronatesters in Furfural umwandelt,
h. Abkühlen der erhitzten zweiten kombinierten Lösung derart, dass die abgekühlte zweite kombinierte Lösung (i) eine zweite wässrige Phase, umfassend Wasser, wasserlösliches Lösungsmittel und Furfural, und (ii) eine zweite nicht wässrige Phase, umfassend wasserunlösliches Lösungsmittel, wasserunlösliche Boronsäure und Furfural, umfasst; und Abscheiden von mindestens einem Anteil der zweiten nicht wässrigen Phase von der gekühlten zweiten kombinierten Lösung; und
i. Rückgewinnen von mindestens einem Anteil des Furfurals in der zweiten nicht wässrigen Phase.

2. Verfahren zum Produzieren von Furfural, umfassend:
a. Bereitstellen einer xylosehaltigen Lösung, umfassend Xylose in einer Menge von größer als oder gleich 0,5 Gew.-%, wobei die xylosehaltige Lösung eine wässrige Lösung ist;
b. Bereitstellen einer Extraktionslösung, umfassend eine wasserunlösliche Boronsäure (BA: R- B(OH)₂) und ein wasserunlösliches Lösungsmittel;
c. Kombinieren der xylosehaltigen Lösung mit der Extraktionslösung, um eine erste kombinierte Lösung bereitzustellen, wobei das Verhältnis von Boronsäure zu Xylose in der ersten kombinierten Lösung jeweils größer als 1:1 molar ist, und wobei die erste kombinierte Lösung eine erste wässrige Phase und eine erste nicht wässrige Phase umfasst, die nicht wässrige Phase umfassend mindestens einen Anteil der Xylose als Xylose-Diboronatester (BA₂X);
d. Abscheiden von mindestens einem Anteil der ersten nicht wässrigen Phase von der ersten kombinierten Lösung;
e. Bereitstellen einer Umwandlungslösung, umfassend ein wasserlösliches Lösungsmittel und Wasser, wobei die Umwandlungslösung einen pH-Wert von kleiner als oder gleich 4 aufweist;
f. Kombinieren von mindestens einem Anteil der nicht wässrigen Phase aus (d) mit der Umwandlungslösung in einem Verhältnis von Umwandlungslösung zu der nicht wässrigen Phase in einem Bereich von jeweils 0,1 bis 10,0 nach Gewicht, um eine zweite kombinierte Lösung auszubilden;
g. Erhitzen der zweiten kombinierten Lösung auf eine Temperatur von größer als oder gleich 100 °C, um mindestens einen Anteil des Xylose-Diboronatesters in Furfural umzuwandeln, wobei die erhitzte zweite kombinierte Lösung eine zweite wässrige Phase, umfassend Wasser, mindestens einen Anteil des wasserlöslichen Lösungsmittels aus der Umwandlungslösung und Furfural und (ii) eine zweite nicht wässrige Phase, umfassend mindestens einen Anteil des wasserunlöslichen Lösungsmittels und der wasserunlöslichen Boronsäure aus der Extraktionslösung und Furfural, umfasst,
h. Abscheiden von mindestens einem Anteil der zweiten nicht wässrigen Phase von der gekühlten zweiten kombinierten Lösung; und
i. Rückgewinnen von mindestens einem Anteil des Furfurals aus der nicht wässrigen Phase.

3. Verfahren nach einem der Ansprüche 1 bis 2, wobei Schritt (i) das Bereitstellen von mindestens einem Anteil der zweiten nicht wässrigen Phase aus (h) an einen Destillationsprozess umfasst, um ein Kopfprodukt, umfassend Furfural, und ein Bodenprodukt, umfassend ein wasserunlösliches Lösungsmittel und wasserunlösliche Boronsäure, zurückzugewinnen.

4. Verfahren nach Anspruch 3, ferner umfassend das Bereitstellen von mindestens einem Anteil des Bodenprodukts zur Verwendung als Teil der Extraktionslösung.

5. Verfahren nach Anspruch 4, wobei mindestens ein Anteil der ersten wässrigen Phase in (c) wasserlösliches Lösungsmittel, wasserunlösliches Lösungsmittel und wasserunlösliche Boronsäure umfasst, das Verfahren ferner umfassend:
Abscheiden von mindestens einem Anteil der ersten wässrigen Phase in (c) von der ersten kombinierten Lösung; und
weiteres Verarbeiten von mindestens einem Anteil der abgeschiedenen ersten wässrigen Phase, um mindestens eines von wasserlöslichem Lösungsmittel,
wasserunlöslichem Lösungsmittel und wasserunlöslicher Boronsäure zurückzugewinnen.

6. Verfahren nach einem der Ansprüche 1 bis 5, ferner umfassend das Durchführen von mindestens einem Anteil der Schritte (c) und (d) in einer Flüssig-Flüssig-Extraktionseinheit in einem Gegenstrombetrieb, wobei die xylosehaltige Lösung bei einer höheren Temperatur als die Temperatur der Extraktionslösung bereitgestellt wird.

7. Verfahren nach einem der Ansprüche 1 bis 6, ferner umfassend das Bereitstellen von mindestens einem Anteil der zweiten wässrigen Phase aus (h) an einen Destillationsprozess, um ein Kopfprodukt, umfassend Wasser und Furfural, und ein saures Bodenprodukt, umfassend Wasser und ein wasserlösliches Lösungsmittel, zurückzugewinnen, wobei das Bodenprodukt einen pH-Wert von weniger als 7 aufweist.

8. Verfahren nach Anspruch 7, ferner umfassend das Bereitstellen von mindestens einem Anteil des sauren Bodenprodukts zur Verwendung als Teil der Umwandlungslösung.

9. Verfahren nach einem der Ansprüche 3 bis 6, ferner umfassend das Bereitstellen von mindestens einem Anteil der zweiten wässrigen Phase aus (h) an einen Lösungsmittelextraktionsprozess, um Furfural zurückzugewinnen, wobei mindestens ein Anteil des Lösungsmittels, das in dem Extraktionsprozess verwendet wird, das Destillationsbodenprodukt umfasst, umfassend wasserunlösliches Lösungsmittel und wasserunlösliche Boronsäure.

10. Verfahren nach einem der Ansprüche 1 bis 9, wobei die xylosehaltige Lösung ein Hydrolysat ist.

11. Verfahren nach einem der Ansprüche 1 bis 10, wobei die wasserunlösliche Boronsäure bei 20 °C eine Löslichkeit in Wasser von bis zu 5 Gew.-% aufweist.

12. Verfahren nach einem der Ansprüche 1 bis 11, wobei die wasserunlösliche Boronsäure aus der Gruppe ausgewählt ist, bestehend aus Phenylboronsäure, 4-Biphenylboronsäure, 4-Butylphenylboronsäure, 4-tert-Butylphenylboronsäure, 4-Ethylphenylboronsäure, 2-Naphthylboronsäure, Naphthalin-1-boronsäure, o-Tolylboronsäure, m-Tolylboronsäure, (2-Methylpropyl)boronsäure, Butylboronsäure, Octylboronsäure, Phenethylboronsäure, Cyclohexylboronsäure und einer beliebigen Kombination davon.

13. Verfahren nach einem der Ansprüche 1 bis 12, wobei das wasserunlösliche Lösungsmittel bei 20 °C eine Löslichkeit in Wasser von bis zu 5 Gew.-% aufweist.

14. Verfahren nach einem der Ansprüche 1 bis 13, wobei das wasserunlösliche Lösungsmittel aus der Gruppe ausgewählt ist, bestehend aus Benzoesäure, Kresol (m), Diisopropylether, Terephthalsäure, Diethylenglykoldiethylether, Anisol, Salicylsäure, 2,6-Xylenol, 4Et-Phenol, Toluol, Benzofuran, Ethylbenzol, Octansäure, 1-Methylnaphthalin, Nitrobenzol, Guajacol, Heptan, 1-Octanol und Methylisobutylketonen oder einer beliebigen Kombination davon.

15. Verfahren nach einem der Ansprüche 1 bis 14, wobei mindestens eines der wasserunlöslichen Boronsäure und des wasserunlöslichen Lösungsmittels einen Siedepunkt aufweist, der höher als der von Furfural ist, vorzugsweise mindestens 5 °C höher.

16. Verfahren nach einem der Ansprüche 1 bis 15, wobei das wasserlösliche Lösungsmittel einen logP (Octanol-Wasser-Verteilungskoeffizient) in einem Bereich von (-3) bis 0 aufweist.

17. Verfahren nach einem der Ansprüche 1 bis 16, wobei das wasserlösliche Lösungsmittel aus der Gruppe ausgewählt ist, bestehend aus Dimethylsulfoxid (DMSO), Diglym, Sulfolan, Gamma-Butyrolacton, Bernsteinsäure, nMe-Acetamid, Dioxan, nMe-Pyrrolidon, Gamma-Valerolacton, Aceton, Essigsäure und einer beliebigen Kombination davon.

18. Verfahren nach einem der Ansprüche 1 bis 17, wobei das wasserlösliche Lösungsmittel einen Siedepunkt aufweist, der höher als der von Wasser ist, vorzugsweise mindestens 5 °C höher.

## Revendications

1. Procédé de production de furfural comprenant :
a. la fourniture d'une solution contenant du xylose comprenant du xylose en une quantité supérieure ou égale à 0,5 % en poids, dans lequel la solution contenant du xylose est une solution aqueuse ;
b. la fourniture d'une solution d'extraction comprenant un acide boronique non hydrosoluble (BA : R-B(OH)₂) et un solvant non hydrosoluble ;
c. la combinaison de la solution contenant du xylose avec la solution d'extraction pour fournir une première solution combinée, dans lequel le rapport entre l'acide boronique et le xylose dans la première solution combinée est supérieur à 1:1 molaire, respectivement, et dans lequel la première solution combinée comprend une première phase aqueuse et une première phase non aqueuse, ladite phase non aqueuse comprenant au moins une partie du xylose en tant que xylose-ester de diboronate (BA₂X) ;
d. la séparation d'au moins une partie de la première phase non aqueuse de la première solution combinée ;
e. la fourniture d'une solution de conversion comprenant un solvant hydrosoluble et de l'eau, ladite solution de conversion ayant un pH inférieur ou égal à 4 ;
f. la combinaison d'au moins une partie de la phase non aqueuse obtenue en (d) avec la solution de conversion dans un rapport entre la solution de conversion et la phase non aqueuse dans une plage de 0,1 à 10,0 en poids, respectivement, pour former une seconde solution combinée ;
g. le chauffage de la seconde solution combinée à une température Tₕ à laquelle ou au-dessus de laquelle la seconde solution combinée est constituée sensiblement d'une phase liquide homogène, dans lequel un tel chauffage convertit au moins une partie du xylose-ester de diboronate en furfural,
h. le refroidissement de la seconde solution combinée chauffée de telle sorte que la seconde solution combinée refroidie comprend (i) une seconde phase aqueuse comprenant de l'eau, un solvant hydrosoluble, et du furfural et (ii) une seconde phase non aqueuse comprenant un solvant non hydrosoluble, un acide boronique non hydrosoluble, et du furfural ; et la séparation d'au moins une partie de la seconde phase non aqueuse de la seconde solution combinée refroidie ; et
i. la récupération d'au moins une partie du furfural dans la seconde phase non aqueuse.

2. Procédé de production de furfural comprenant :
a. la fourniture d'une solution contenant du xylose comprenant du xylose en une quantité supérieure ou égale à 0,5 % en poids, dans lequel la solution contenant du xylose est une solution aqueuse ;
b. la fourniture d'une solution d'extraction comprenant un acide boronique non hydrosoluble (BA : R-B(OH)₂) et un solvant non hydrosoluble ;
c. la combinaison de la solution contenant du xylose avec la solution d'extraction pour fournir une première solution combinée, dans lequel le rapport entre l'acide boronique et le xylose dans la première solution combinée est supérieur à 1:1 molaire, respectivement, et dans lequel la première solution combinée comprend une première phase aqueuse et une première phase non aqueuse, ladite phase non aqueuse comprenant au moins une partie du xylose en tant que xylose-ester de diboronate (BA₂X) ;
d. la séparation d'au moins une partie de la première phase non aqueuse de la première solution combinée ;
e. la fourniture d'une solution de conversion comprenant un solvant hydrosoluble et de l'eau, ladite solution de conversion ayant un pH inférieur ou égal à 4 ;
f. la combinaison d'au moins une partie de la phase non aqueuse obtenue en (d) avec la solution de conversion dans un rapport entre la solution de conversion et la phase non aqueuse dans une plage de 0,1 à 10,0 en poids, respectivement, pour former une seconde solution combinée ;
g. le chauffage de la seconde solution combinée à une température supérieure ou égale à 100 °C pour convertir au moins une partie du xylose-ester de diboronate en furfural, dans lequel la seconde solution combinée chauffée comprend une seconde phase aqueuse comprenant de l'eau, au moins une partie du solvant hydrosoluble de la solution de conversion, et du furfural et (ii) une seconde phase non aqueuse comprenant au moins une partie du solvant non hydrosoluble et de l'acide boronique non hydrosoluble de la solution d'extraction, et du furfural,
h. la séparation d'au moins une partie de la seconde phase non aqueuse de la seconde solution combinée refroidie ; et
i. la récupération d'au moins une partie du furfural de la phase non aqueuse.

3. Procédé selon l'une quelconque des revendications 1 à 2 dans lequel l'étape (i) comprend la fourniture d'au moins une partie de la seconde phase non aqueuse de (h) à un procédé de distillation pour récupérer un produit de tête comprenant du furfural et un produit de fond comprenant un solvant non hydrosoluble et un acide boronique non hydrosoluble.

4. Procédé selon la revendication 3 comprenant en outre la fourniture d'au moins une partie du produit de fond pour l'utiliser en tant que partie de la solution d'extraction.

5. Procédé selon la revendication 4 dans lequel au moins une partie de la première phase aqueuse en (c) comprend un solvant hydrosoluble, un solvant non hydrosoluble, et un acide boronique non hydrosoluble, ledit procédé comprenant en outre :
la séparation d'au moins une partie de la première phase aqueuse en (c) de la première solution combinée ; et
le traitement supplémentaire d'au moins une partie de la première phase aqueuse séparée pour récupérer au moins l'un parmi un solvant hydrosoluble, un solvant non hydrosoluble, et un acide boronique non hydrosoluble.

6. Procédé selon l'une quelconque des revendications 1 à 5 comprenant en outre la réalisation d'au moins une partie des étapes (c) et (d) dans une unité d'extraction liquide-liquide en fonctionnement à contre-courant, dans lequel la solution contenant du xylose est fournie à une température plus élevée que la température de la solution d'extraction.

7. Procédé selon l'une quelconque des revendications 1 à 6 comprenant en outre la fourniture d'au moins une partie de la seconde phase aqueuse de (h) à un procédé de distillation pour récupérer un produit de tête comprenant de l'eau et du furfural et un produit de fond acide comprenant de l'eau et un solvant hydrosoluble, dans lequel le produit de fond a un pH inférieur à 7.

8. Procédé selon la revendication 7 comprenant en outre la fourniture d'au moins une partie du produit de fond acide pour l'utiliser en tant que partie de la solution de conversion.

9. Procédé selon l'une quelconque des revendications 3 à 6 comprenant en outre la fourniture d'au moins une partie de la seconde phase aqueuse de (h) à un procédé d'extraction par solvant pour récupérer du furfural, dans lequel au moins une partie du solvant utilisé dans le procédé d'extraction comprend le produit de fond de distillation comprenant un solvant non hydrosoluble et un acide boronique non hydrosoluble.

10. Procédé selon l'une quelconque des revendications 1 à 9 dans lequel la solution contenant du xylose est un hydrolysat.

11. Procédé selon l'une quelconque des revendications 1 à 10 dans lequel l'acide boronique non hydrosoluble présente une hydrosolubilité à 20 °C jusqu'à 5 % en poids.

12. Procédé selon l'une quelconque des revendications 1 à 11 dans lequel l'acide boronique non hydrosoluble est choisi dans le groupe constitué d'acide phénylboronique, acide 4-biphénylboronique, acide 4-butylphénylboronique, acide 4-tert-butylphénylboronique, acide 4-éthylphénylboronique, acide 2-naphtylboronique, acide naphtalène-1-boronique, acide o-tolylboronique, acide m-tolylboronique, acide (2-méthylpropyl)boronique, acide butylboronique, acide octylboronique, acide phénéthylboronique, acide cyclohexylboronique, et une combinaison quelconque de ceux-ci.

13. Procédé selon l'une quelconque des revendications 1 à 12 dans lequel le solvant non hydrosoluble présente une hydrosolubilité à 20 °C jusqu'à 5 % en poids.

14. Procédé selon l'une quelconque des revendications 1 à 13, dans lequel le solvant non hydrosoluble est choisi dans le groupe constitué d'acide benzoïque, crésol (m), éther di-isopropylique, acide téréphtalique, éther diéthylique du diéthylène glycol, anisole, acide salicylique, 2,6-xylénol, 4Et-phénol, toluène, benzofurane, éthylbenzène, acide octanoïque, 1-méthylnaphtalène, nitrobenzène, gaïacol, heptane, 1-octanol et méthylisobutylcétones, et une combinaison quelconque de ceux-ci.

15. Procédé selon l'une quelconque des revendications 1 à 14 dans lequel au moins l'un de l'acide boronique non hydrosoluble et du solvant non hydrosoluble a un point d'ébullition supérieur à celui du furfural, de préférence supérieur d'au moins 5 °C.

16. Procédé selon l'une quelconque des revendications 1 à 15 dans lequel le solvant hydrosoluble a un logP (coefficient de partage octanol/eau) dans une plage de (-3) à 0.

17. Procédé selon l'une quelconque des revendications 1 à 16 dans lequel le solvant hydrosoluble est choisi dans le groupe constitué de diméthylsulfoxyde (DMSO), diglyme, sulfolane, gamma-butyrolactone, acide succinique, nMe-acétamide, dioxane, nMe-pyrrolidone, gamma valérolactone, acétone, acide acétique, et une combinaison quelconque de ceux-ci.

18. Procédé selon l'une quelconque des revendications 1 à 17 dans lequel le solvant hydrosoluble a un point d'ébullition supérieur à celui de l'eau, de préférence supérieur d'au moins 5 °C.
